# EUROPEAN PATENT APPLICATION

(11) **EP 3 862 437 A1**
(43) Date of publication of application: **11.08.2021**
(21) Application number: 19869215.4
(22) Date of filing: 26.09.2019
(51) Int. Cl.: C12Q 1/37, C12N 9/64, G01N 33/569, G01N 33/579, C12N 15/57

(54) **METHOD FOR ENHANCING SENSITIVITY OF ENDOTOXIN MEASURING AGENT**

(30) Priority: 03.10.2018 JP 2018188587
(71) Applicant: Seikagaku Corporation, Tokyo 100-0005 (JP)
(72) Inventor: OGURA, Norihiko, Tokyo 100-0005 (JP); KOBAYASHI, Yuki, Tokyo 100-0005 (JP); MIZUMURA, Hikaru, Tokyo 100-0005 (JP); ODA, Toshio, Tokyo 100-0005 (JP)
(74) Representative: Müller-Boré & Partner Patentanwälte PartG mbB
(86) International application number: PCT/JP2019/037828
(87) International publication number: WO 2020/071229

(57) **Abstract**

An object of the present invention is to provide a method for enhancing a sensitivity of a current endotoxin measuring reagent employing a recombinant protein to the endotoxin of Helicobacter pylori. The present invention provides a method for enhancing the sensitivity of an endotoxin measuring reagent to the endotoxin of Helicobacter pylori, the endotoxin measuring reagent containing a recombinant protein of horseshoe crab factor C, the method including increasing a content of the recombinant protein of factor C at the time of endotoxin measurement to an amount that is sufficient for enhancing the sensitivity.

SEQUENCE LISTING

<110>  Seikagaku Corporation

<120>
\203G\203\223\203h\203g\203L\203V\203\223\221ª\222è\215Ü\202Ì\212´\223x\202Ì
\221\235\213-\225û\226@

<130>  F201905-9296

<150>  JP2018-188587
<151>  2018-10-03

<160>  12

<170>  PatentIn version 3.5

<210>  1
<211>  3060
<212>  DNA
<213>  Tachypleus tridentatus

<220>
<223>  Tachypleus tridentatus factor C

<400>  1
atggtcttag cgtcgttttt ggtgtctggt ttagttctag ggatactagc ccaacaaatg      60
cgtccagttc agtccagagg agtagatctg ggcttgtgtg atgaaacgag gttcgagtgt     120
aagtgtggag atccaggcta tgtgttcaac gtccctatga aacaatgcac gtacttctat     180
cgatggaggc cttattgtaa accatgtgat gacctggagg ctaaggacat ttgtccaaag     240
tacaaacgat gtcaagagtg taaggctggt cttgatagtt gtgttacttg tccacctaac     300
aaatatggta cttggtgtag cggtgaatgt caatgtaaga atggaggtat ctgtgaccag     360
aggacaggag cttgtacctg tcgtgacaga tatgaaggag cgcactgtga aattctcaaa     420
ggttgtcctc ttcttccatc ggattctcaa gttcaggaag tcagaaaccc accagataat     480
ccccaaacta ttgactacag ctgttcacca gggttcaagc ttaaaggcgt ggcacgaatt     540
agctgtctcc caaatggaca gtggagtagc tttccaccca aatgtattcg agaatgtgcc     600
aaggtttcat ctccagaaca cgggaaagtg aatgctccta gtggcaatat gatagaaggg     660
gctactttac ggttctcatg tgatagtccc tactacttga ttggtcaaga aacattaacc     720
tgccagggta atggtcagtg gagtggacaa ataccacaat gtaagaagtt ggtcttctgt     780
cctgaccttg atcctgtaaa ccatgctgaa caccaggtta aaattggtgt ggaacaaaaa     840
tatggtcagt ttcctcaagg cactgaagtg acctatacgt gttcgggtaa ctacttcttg     900
atgggtttta acaccttaaa atgtaaccct gatgggtcct ggtcaggatc acagccatcc     960
tgtgttaaag tggcagacag agaggtcgac tgtgacagta aagctgtaga cttcttggat    1020
gatgttggtg aacctgtcag gatccactgt cctgctggct gttctttgac agctggtact    1080
gtgtggggta cagccatata ccacgaactt tcctcagtgt gtcgtgcagc catccatgct    1140
ggcaagcttc caaactctgg aggggcggtg catgtagtga acaatggccc ctactcggac    1200
tttctgggta gtgacctgaa tgggataaaa tcggaagagt tgaagtctct tgcccgcagt    1260
tttcgatttg attatgtcag ttcatccaca gcaggtagat caggatgtcc tgatggatgg    1320
tttgaggtag aagagaactg tgtgtacgtt acatcaaaac agagagcctg ggaaagagct    1380
caaggtgtgt gtaccaatat ggctgctcgt cttgctgtgc tagacaaaga tctaattccg    1440
agttccttga ctgagactct acgagggaaa gggttaacaa ccacatggat aggattgcac    1500
agactagatg ctgagaagcc ctttgtttgg gagctaatgg atcgtagtaa tgtggttctg    1560
aatgataacc taacattctg ggcctctggc gaacctggaa atgaaactaa ctgtgtatat    1620
ctggacatcc gagatcagct gcagcctgtg tggaaaacca agtcatgttt tcagccctca    1680
agctttgctt gcatgatgga tttgtcagac agaaataaag ccaaatgcga tgaccctgga    1740
ccactggaaa atggacacgc cacacttcat ggacaaagta ttgatgggtt ctatgctggt    1800
tcttctataa ggtacagctg tgaggttctc cactacctca gtggaactga gaccgtaact    1860
tgtacaacaa atggcacatg gagtgctcct aaacctcgat gtatcaaagt catcacctgc    1920
caaaaccctc ctgtaccatc atatggttct gtggaaatca aacccccaag tcggacaaac    1980
tcgatcagtc gtgttgggtc acctttcttg aggttgccac ggttacccct cccattagcc    2040
agagcagcca aacctcctcc aaaacctaga tcctcacaac cctctactgt ggacttggct    2100
tctaaagtta aactacctga aggtcattac cgggtagggt ctcgagccat ttacacgtgc    2160
gagtcgagat actacgaact acttggatct caaggcagaa gatgtgactc taatggaaac    2220

tggagtggtc ggcccgctag ctgtattcca gtttgtggac ggtcagactc tcctcgttct 2280
cctttcatct ggaatgggaa ttctacagaa ataggtcagt ggccgtggca ggcaggaatc 2340
tctcgatggc ttgcagacca caatatgtgg tttctccagt gtggaggatc cctattgaat 2400
gagaaatgga tcgtcactgc tgcccactgt gtcacctact ctgctactgc tgagataatt 2460
gatcccagtc agtttaaaat ctatctgggc aagtactacc gtgatgacag tagagacgat 2520
gactacgtac aagtaagaga ggctctcgag atccacgtaa atcctaacta cgaccccggc 2580
aatctcaact ttgacatagc cctaattcaa ctgaaaactc ctgttacttt gacaacacga 2640
gtccaaccaa tctgtctgcc tactgacatc acaacaagag aacacttgaa ggagggaaca 2700
ttagcagtgg tgacaggttg gggtttgaat gaaaacaaca catattcaga gatgattcaa 2760
caagctgtgc tacctgttgt tgcagcaagc acctgtgaag aggggtacaa ggaagcagac 2820
ttaccactga cagtaacaga gaacatgttc tgtgcaggtt acaagaaggg acgttatgat 2880
gcctgcagtg gggacagtgg aggaccatta gtgtttgctg atgattcccg taccgaaagg 2940
cggtgggtct tggaagggat tgtcagctgg ggcagtccca gtggatgtgg caaggctaac 3000
cagtatgggg gcttcactaa agttaacgtt tttctatcat ggattaggca gttcatttga 3060

<210> 2
<211> 1019
<212> PRT
<213> Tachypleus tridentatus

<220>
<223> Tachypleus tridentatus factor C

<400> 2
Met Val Leu Ala Ser Phe Leu Val Ser Gly Leu Val Leu Gly Ile Leu
1               5                   10                  15
Ala Gln Gln Met Arg Pro Val Gln Ser Arg Gly Val Asp Leu Gly Leu
            20                  25                  30
Cys Asp Glu Thr Arg Phe Glu Cys Lys Cys Gly Asp Pro Gly Tyr Val
        35                  40                  45
Phe Asn Val Pro Met Lys Gln Cys Thr Tyr Phe Tyr Arg Trp Arg Pro
    50                  55                  60
Tyr Cys Lys Pro Cys Asp Asp Leu Glu Ala Lys Asp Ile Cys Pro Lys
65                  70                  75                  80
Tyr Lys Arg Cys Gln Glu Cys Lys Ala Gly Leu Asp Ser Cys Val Thr
                85                  90                  95
Cys Pro Pro Asn Lys Tyr Gly Thr Trp Cys Ser Gly Glu Cys Gln Cys
            100                 105                 110
Lys Asn Gly Gly Ile Cys Asp Gln Arg Thr Gly Ala Cys Thr Cys Arg
        115                 120                 125
Asp Arg Tyr Glu Gly Ala His Cys Glu Ile Leu Lys Gly Cys Pro Leu
    130                 135                 140
Leu Pro Ser Asp Ser Gln Val Gln Glu Val Arg Asn Pro Pro Asp Asn
145                 150                 155                 160
Pro Gln Thr Ile Asp Tyr Ser Cys Ser Pro Gly Phe Lys Leu Lys Gly
                165                 170                 175
Val Ala Arg Ile Ser Cys Leu Pro Asn Gly Gln Trp Ser Ser Phe Pro
            180                 185                 190
Pro Lys Cys Ile Arg Glu Cys Ala Lys Val Ser Ser Pro Glu His Gly
        195                 200                 205
Lys Val Asn Ala Pro Ser Gly Asn Met Ile Glu Gly Ala Thr Leu Arg
    210                 215                 220
Phe Ser Cys Asp Ser Pro Tyr Tyr Leu Ile Gly Gln Glu Thr Leu Thr
225                 230                 235                 240
Cys Gln Gly Asn Gly Gln Trp Ser Gly Gln Ile Pro Gln Cys Lys Lys
                245                 250                 255
Leu Val Phe Cys Pro Asp Leu Asp Pro Val Asn His Ala Glu His Gln
            260                 265                 270
Val Lys Ile Gly Val Glu Gln Lys Tyr Gly Gln Phe Pro Gln Gly Thr
        275                 280                 285
Glu Val Thr Tyr Thr Cys Ser Gly Asn Tyr Phe Leu Met Gly Phe Asn

290                 295                 300
Thr Leu Lys Cys Asn Pro Asp Gly Ser Trp Ser Gly Ser Gln Pro Ser
305                 310                 315                 320
Cys Val Lys Val Ala Asp Arg Glu Val Asp Cys Asp Ser Lys Ala Val
                325                 330                 335
Asp Phe Leu Asp Asp Val Gly Glu Pro Val Arg Ile His Cys Pro Ala
            340                 345                 350
Gly Cys Ser Leu Thr Ala Gly Thr Val Trp Gly Thr Ala Ile Tyr His
        355                 360                 365
Glu Leu Ser Ser Val Cys Arg Ala Ala Ile His Ala Gly Lys Leu Pro
    370                 375                 380
Asn Ser Gly Gly Ala Val His Val Val Asn Asn Gly Pro Tyr Ser Asp
385                 390                 395                 400
Phe Leu Gly Ser Asp Leu Asn Gly Ile Lys Ser Glu Glu Leu Lys Ser
                405                 410                 415
Leu Ala Arg Ser Phe Arg Phe Asp Tyr Val Ser Ser Ser Thr Ala Gly
            420                 425                 430
Arg Ser Gly Cys Pro Asp Gly Trp Phe Glu Val Glu Glu Asn Cys Val
        435                 440                 445
Tyr Val Thr Ser Lys Gln Arg Ala Trp Glu Arg Ala Gln Gly Val Cys
    450                 455                 460
Thr Asn Met Ala Ala Arg Leu Ala Val Leu Asp Lys Asp Leu Ile Pro
465                 470                 475                 480
Ser Ser Leu Thr Glu Thr Leu Arg Gly Lys Gly Leu Thr Thr Thr Trp
                485                 490                 495
Ile Gly Leu His Arg Leu Asp Ala Glu Lys Pro Phe Val Trp Glu Leu
            500                 505                 510
Met Asp Arg Ser Asn Val Val Leu Asn Asp Asn Leu Thr Phe Trp Ala
        515                 520                 525
Ser Gly Glu Pro Gly Asn Glu Thr Asn Cys Val Tyr Leu Asp Ile Arg
    530                 535                 540
Asp Gln Leu Gln Pro Val Trp Lys Thr Lys Ser Cys Phe Gln Pro Ser
545                 550                 555                 560
Ser Phe Ala Cys Met Met Asp Leu Ser Asp Arg Asn Lys Ala Lys Cys
                565                 570                 575
Asp Asp Pro Gly Pro Leu Glu Asn Gly His Ala Thr Leu His Gly Gln
            580                 585                 590
Ser Ile Asp Gly Phe Tyr Ala Gly Ser Ser Ile Arg Tyr Ser Cys Glu
        595                 600                 605
Val Leu His Tyr Leu Ser Gly Thr Glu Thr Val Thr Cys Thr Thr Asn
    610                 615                 620
Gly Thr Trp Ser Ala Pro Lys Pro Arg Cys Ile Lys Val Ile Thr Cys
625                 630                 635                 640
Gln Asn Pro Pro Val Pro Ser Tyr Gly Ser Val Glu Ile Lys Pro Pro
                645                 650                 655
Ser Arg Thr Asn Ser Ile Ser Arg Val Gly Ser Pro Phe Leu Arg Leu
            660                 665                 670
Pro Arg Leu Pro Leu Pro Leu Ala Arg Ala Ala Lys Pro Pro Pro Lys
        675                 680                 685
Pro Arg Ser Ser Gln Pro Ser Thr Val Asp Leu Ala Ser Lys Val Lys
    690                 695                 700
Leu Pro Glu Gly His Tyr Arg Val Gly Ser Arg Ala Ile Tyr Thr Cys
705                 710                 715                 720
Glu Ser Arg Tyr Tyr Glu Leu Leu Gly Ser Gln Gly Arg Arg Cys Asp
                725                 730                 735
Ser Asn Gly Asn Trp Ser Gly Arg Pro Ala Ser Cys Ile Pro Val Cys
            740                 745                 750
Gly Arg Ser Asp Ser Pro Arg Ser Pro Phe Ile Trp Asn Gly Asn Ser
        755                 760                 765
Thr Glu Ile Gly Gln Trp Pro Trp Gln Ala Gly Ile Ser Arg Trp Leu
    770                 775                 780
Ala Asp His Asn Met Trp Phe Leu Gln Cys Gly Gly Ser Leu Leu Asn
785                 790                 795                 800

Glu Lys Trp Ile Val Thr Ala Ala His Cys Val Thr Tyr Ser Ala Thr
                805                 810                 815
Ala Glu Ile Ile Asp Pro Ser Gln Phe Lys Ile Tyr Leu Gly Lys Tyr
            820                 825                 830
Tyr Arg Asp Asp Ser Arg Asp Asp Asp Tyr Val Gln Val Arg Glu Ala
        835                 840                 845
Leu Glu Ile His Val Asn Pro Asn Tyr Asp Pro Gly Asn Leu Asn Phe
    850                 855                 860
Asp Ile Ala Leu Ile Gln Leu Lys Thr Pro Val Thr Leu Thr Thr Arg
865                 870                 875                 880
Val Gln Pro Ile Cys Leu Pro Thr Asp Ile Thr Thr Arg Glu His Leu
                885                 890                 895
Lys Glu Gly Thr Leu Ala Val Val Thr Gly Trp Gly Leu Asn Glu Asn
            900                 905                 910
Asn Thr Tyr Ser Glu Met Ile Gln Gln Ala Val Leu Pro Val Val Ala
        915                 920                 925
Ala Ser Thr Cys Glu Glu Gly Tyr Lys Glu Ala Asp Leu Pro Leu Thr
    930                 935                 940
Val Thr Glu Asn Met Phe Cys Ala Gly Tyr Lys Lys Gly Arg Tyr Asp
945                 950                 955                 960
Ala Cys Ser Gly Asp Ser Gly Gly Pro Leu Val Phe Ala Asp Asp Ser
                965                 970                 975
Arg Thr Glu Arg Arg Trp Val Leu Glu Gly Ile Val Ser Trp Gly Ser
            980                 985                 990
Pro Ser Gly Cys Gly Lys Ala Asn  Gln Tyr Gly Gly Phe  Thr Lys Val
        995                 1000                1005
Asn Val  Phe Leu Ser Trp Ile  Arg Gln Phe Ile
    1010                1015

<210>  3
<211>  3060
<212>  DNA
<213>  Carcinoscorpius rotundicauda

<220>
<223>  Carcinoscorpius rotundicauda factor C

<400>  3
   atggtcttag cgtcgttttt ggtgtctggt ttagttctag ggctactagc ccaaaaaatg      60
   cgcccagttc agtccaaagg agtagatcta ggcttgtgtg atgaaacgag gttcgagtgt     120
   aagtgtggcg atccaggcta tgtgttcaac attccagtga aacaatgtac atacttttat     180
   cgatggaggc cgtattgtaa accatgtgat gacctggagg ctaaggatat ttgtccaaag     240
   tacaaacgat gtcaagagtg taaggctggt cttgatagtt gtgttacttg tccacctaac     300
   aaatatggta cttggtgtag cggtgaatgt cagtgtaaga atggaggtat ctgtgaccag     360
   aggacaggag cttgtgcatg tcgtgacaga tatgaagggg tgcactgtga aattctcaaa     420
   ggttgtcctc ttcttccatc ggattctcag gttcaggaag tcagaaatcc accagataat     480
   ccccaaacta ttgactacag ctgttcacca gggttcaagc ttaagggtat ggcacgaatt     540
   agctgtctcc caaatggaca gtggagtaac tttccaccca aatgtattcg agaatgtgcc     600
   atggtttcat ctccagaaca tgggaaagtg aatgctctta gtggtgatat gatagaaggg     660
   gctactttac ggttctcatg tgatagtccc tactacttga ttggtcaaga aacattaacc     720
   tgtcagggta atggtcagtg gaatggacag ataccacaat gtaagaactt ggtcttctgt     780
   cctgacctgg atcctgtaaa ccatgctgaa cacaaggtta aaattggtgt ggaacaaaaa     840
   tatggtcagt ttcctcaagg cactgaagtg acctatacgt gttcgggtaa ctacttcttg     900
   atgggttttg acaccttaaa atgtaaccct gatgggtctt ggtcaggatc acagccatcc     960
   tgtgttaaag tggcagacag agaggtcgac tgtgacagta aagctgtaga cttcttggat    1020
   gatgttggtg aacctgtcag gatccactgt cctgctggct gttctttgac agctggtact    1080
   gtgtggggta cagccatata ccatgaactt tcctcagtgt gtcgtgcagc catccatgct    1140
   ggcaagcttc caaactctgg aggagcggtg catgttgtga acaatggccc ctactcggac    1200
   tttctgggta gtgacctgaa tgggataaaa tcggaagagt tgaagtctct tgcccggagt    1260
   ttccgattcg attatgtccg ttcctccaca gcaggtaaat caggatgtcc tgatggatgg    1320
   tttgaggtag acgagaactg tgtgtacgtt acatcaaaac agagagcctg ggaaagagct    1380
   caaggtgtgt gtaccaatat ggctgctcgt cttgctgtgc tggacaaaga tgtaattcca    1440
   aattcgttga ctgagactct acgagggaaa gggttaacaa ccacgtggat aggattgcac 1500
   agactagatg ctgagaagcc ctttatttgg gagttaatgg atcgtagtaa tgtggttctg 1560
   aatgataacc taacattctg ggcctctggc gaacctggaa atgaaactaa ctgtgtatat 1620
   atggacatcc aagatcagtt gcagtctgtg tggaaaacca agtcatgttt tcagccctca 1680
   agttttgctt gcatgatgga tctgtcagac agaaataaag ccaaatgcga tgatcctgga 1740
   tcactggaaa atggacacgc cacacttcat ggacaaagta ttgatgggtt ctatgctggt 1800
   tcttctataa ggtacagctg tgaggttctc cactacctca gtggaactga aaccgtaact 1860
   tgtacaacaa atggcacatg gagtgctcct aaacctcgat gtatcaaagt catcacctgc 1920
   caaaaccccc ctgtaccatc atatggttct gtggaaatca aacccccaag tcggacaaac 1980
   tcgataagtc gtgttgggtc acctttcttg aggttgccac ggttacccct cccattagct 2040
   agagcagcca aacctcctcc aaaacctaga tcctcacaac cctctactgt ggacttggct 2100
   tctaaagtta aactacctga aggtcattac cgggtagggt ctcgagccat ctacacgtgc 2160
   gagtcgagat actacgaact acttggatct caaggcagaa gatgtgactc taatggaaac 2220
   tggagtggtc ggccagcgag ctgtattcca gtttgtggac ggtcagactc tcctcgttct 2280
   ccttttatct ggaatgggaa ttctacagaa ataggtcagt ggccgtggca ggcaggaatc 2340
   tctagatggc ttgcagacca caatatgtgg tttctccagt gtggaggatc tctattgaat 2400
   gagaaatgga tcgtcactgc tgcccactgt gtcacctact ctgctactgc tgagattatt 2460
   gaccccaatc agtttaaaat gtatctgggc aagtactacc gtgatgacag tagagacgat 2520
   gactatgtac aagtaagaga ggctcttgag atccacgtga atcctaacta cgaccccggc 2580
   aatctcaact ttgacatagc cctaattcaa ctgaaaactc ctgttacttt gacaacacga 2640
   gtccaaccaa tctgtctgcc tactgacatc acaacaagag aacacttgaa ggagggaaca 2700
   ttagcagtgg tgacaggttg gggtttgaat gaaaacaaca cctattcaga gacgattcaa 2760
   caagctgtgc tacctgttgt tgcagccagc acctgtgaag aggggtacaa ggaagcagac 2820
   ttaccactga cagtaacaga gaacatgttc tgtgcaggtt acaagaaggg acgttatgat 2880
   gcctgcagtg gggacagtgg aggaccttta gtgtttgctg atgattcccg taccgaaagg 2940
   cggtgggtct tggaagggat tgtcagctgg ggcagtccca gtggatgtgg caaggcgaac 3000
   cagtacgggg gcttcactaa agttaacgtt ttcctgtcat ggattaggca gttcatttga 3060

<210> 4
<211> 1019
<212> PRT
<213> Carcinoscorpius rotundicauda

<220>
<223> Carcinoscorpius rotundicauda factor C

<400> 4
Met Val Leu Ala Ser Phe Leu Val Ser Gly Leu Val Leu Gly Leu Leu
1               5                   10                  15
Ala Gln Lys Met Arg Pro Val Gln Ser Lys Gly Val Asp Leu Gly Leu
            20                  25                  30
Cys Asp Glu Thr Arg Phe Glu Cys Lys Cys Gly Asp Pro Gly Tyr Val
        35                  40                  45
Phe Asn Ile Pro Val Lys Gln Cys Thr Tyr Phe Tyr Arg Trp Arg Pro
    50                  55                  60
Tyr Cys Lys Pro Cys Asp Asp Leu Glu Ala Lys Asp Ile Cys Pro Lys
65                  70                  75                  80
Tyr Lys Arg Cys Gln Glu Cys Lys Ala Gly Leu Asp Ser Cys Val Thr
                85                  90                  95
Cys Pro Pro Asn Lys Tyr Gly Thr Trp Cys Ser Gly Glu Cys Gln Cys
            100                 105                 110
Lys Asn Gly Gly Ile Cys Asp Gln Arg Thr Gly Ala Cys Ala Cys Arg
        115                 120                 125
Asp Arg Tyr Glu Gly Val His Cys Glu Ile Leu Lys Gly Cys Pro Leu
    130                 135                 140
Leu Pro Ser Asp Ser Gln Val Gln Glu Val Arg Asn Pro Pro Asp Asn
145                 150                 155                 160
Pro Gln Thr Ile Asp Tyr Ser Cys Ser Pro Gly Phe Lys Leu Lys Gly
                165                 170                 175
Met Ala Arg Ile Ser Cys Leu Pro Asn Gly Gln Trp Ser Asn Phe Pro
            180                 185                 190
Pro Lys Cys Ile Arg Glu Cys Ala Met Val Ser Ser Pro Glu His Gly

195                 200                 205
Lys Val Asn Ala Leu Ser Gly Asp Met Ile Glu Gly Ala Thr Leu Arg
    210                 215                 220
Phe Ser Cys Asp Ser Pro Tyr Tyr Leu Ile Gly Gln Glu Thr Leu Thr
225                 230                 235                 240
Cys Gln Gly Asn Gly Gln Trp Asn Gly Gln Ile Pro Gln Cys Lys Asn
                245                 250                 255
Leu Val Phe Cys Pro Asp Leu Asp Pro Val Asn His Ala Glu His Lys
            260                 265                 270
Val Lys Ile Gly Val Glu Gln Lys Tyr Gly Gln Phe Pro Gln Gly Thr
        275                 280                 285
Glu Val Thr Tyr Thr Cys Ser Gly Asn Tyr Phe Leu Met Gly Phe Asp
    290                 295                 300
Thr Leu Lys Cys Asn Pro Asp Gly Ser Trp Ser Gly Ser Gln Pro Ser
305                 310                 315                 320
Cys Val Lys Val Ala Asp Arg Glu Val Asp Cys Asp Ser Lys Ala Val
                325                 330                 335
Asp Phe Leu Asp Asp Val Gly Glu Pro Val Arg Ile His Cys Pro Ala
            340                 345                 350
Gly Cys Ser Leu Thr Ala Gly Thr Val Trp Gly Thr Ala Ile Tyr His
        355                 360                 365
Glu Leu Ser Ser Val Cys Arg Ala Ala Ile His Ala Gly Lys Leu Pro
    370                 375                 380
Asn Ser Gly Gly Ala Val His Val Val Asn Asn Gly Pro Tyr Ser Asp
385                 390                 395                 400
Phe Leu Gly Ser Asp Leu Asn Gly Ile Lys Ser Glu Glu Leu Lys Ser
                405                 410                 415
Leu Ala Arg Ser Phe Arg Phe Asp Tyr Val Arg Ser Ser Thr Ala Gly
            420                 425                 430
Lys Ser Gly Cys Pro Asp Gly Trp Phe Glu Val Asp Glu Asn Cys Val
        435                 440                 445
Tyr Val Thr Ser Lys Gln Arg Ala Trp Glu Arg Ala Gln Gly Val Cys
    450                 455                 460
Thr Asn Met Ala Ala Arg Leu Ala Val Leu Asp Lys Asp Val Ile Pro
465                 470                 475                 480
Asn Ser Leu Thr Glu Thr Leu Arg Gly Lys Gly Leu Thr Thr Thr Trp
                485                 490                 495
Ile Gly Leu His Arg Leu Asp Ala Glu Lys Pro Phe Ile Trp Glu Leu
            500                 505                 510
Met Asp Arg Ser Asn Val Val Leu Asn Asp Asn Leu Thr Phe Trp Ala
        515                 520                 525
Ser Gly Glu Pro Gly Asn Glu Thr Asn Cys Val Tyr Met Asp Ile Gln
    530                 535                 540
Asp Gln Leu Gln Ser Val Trp Lys Thr Lys Ser Cys Phe Gln Pro Ser
545                 550                 555                 560
Ser Phe Ala Cys Met Met Asp Leu Ser Asp Arg Asn Lys Ala Lys Cys
                565                 570                 575
Asp Asp Pro Gly Ser Leu Glu Asn Gly His Ala Thr Leu His Gly Gln
            580                 585                 590
Ser Ile Asp Gly Phe Tyr Ala Gly Ser Ser Ile Arg Tyr Ser Cys Glu
        595                 600                 605
Val Leu His Tyr Leu Ser Gly Thr Glu Thr Val Thr Cys Thr Thr Asn
    610                 615                 620
Gly Thr Trp Ser Ala Pro Lys Pro Arg Cys Ile Lys Val Ile Thr Cys
625                 630                 635                 640
Gln Asn Pro Pro Val Pro Ser Tyr Gly Ser Val Glu Ile Lys Pro Pro
                645                 650                 655
Ser Arg Thr Asn Ser Ile Ser Arg Val Gly Ser Pro Phe Leu Arg Leu
            660                 665                 670
Pro Arg Leu Pro Leu Pro Leu Ala Arg Ala Ala Lys Pro Pro Pro Lys
        675                 680                 685
Pro Arg Ser Ser Gln Pro Ser Thr Val Asp Leu Ala Ser Lys Val Lys
    690                 695                 700

Leu Pro Glu Gly His Tyr Arg Val Gly Ser Arg Ala Ile Tyr Thr Cys
705                 710                 715                 720
Glu Ser Arg Tyr Tyr Glu Leu Leu Gly Ser Gln Gly Arg Arg Cys Asp
                725                 730                 735
Ser Asn Gly Asn Trp Ser Gly Arg Pro Ala Ser Cys Ile Pro Val Cys
            740                 745                 750
Gly Arg Ser Asp Ser Pro Arg Ser Pro Phe Ile Trp Asn Gly Asn Ser
        755                 760                 765
Thr Glu Ile Gly Gln Trp Pro Trp Gln Ala Gly Ile Ser Arg Trp Leu
    770                 775                 780
Ala Asp His Asn Met Trp Phe Leu Gln Cys Gly Gly Ser Leu Leu Asn
785                 790                 795                 800
Glu Lys Trp Ile Val Thr Ala Ala His Cys Val Thr Tyr Ser Ala Thr
                805                 810                 815
Ala Glu Ile Ile Asp Pro Asn Gln Phe Lys Met Tyr Leu Gly Lys Tyr
            820                 825                 830
Tyr Arg Asp Asp Ser Arg Asp Asp Asp Tyr Val Gln Val Arg Glu Ala
        835                 840                 845
Leu Glu Ile His Val Asn Pro Asn Tyr Asp Pro Gly Asn Leu Asn Phe
    850                 855                 860
Asp Ile Ala Leu Ile Gln Leu Lys Thr Pro Val Thr Leu Thr Thr Arg
865                 870                 875                 880
Val Gln Pro Ile Cys Leu Pro Thr Asp Ile Thr Thr Arg Glu His Leu
                885                 890                 895
Lys Glu Gly Thr Leu Ala Val Val Thr Gly Trp Gly Leu Asn Glu Asn
            900                 905                 910
Asn Thr Tyr Ser Glu Thr Ile Gln Gln Ala Val Leu Pro Val Val Ala
        915                 920                 925
Ala Ser Thr Cys Glu Glu Gly Tyr Lys Glu Ala Asp Leu Pro Leu Thr
    930                 935                 940
Val Thr Glu Asn Met Phe Cys Ala Gly Tyr Lys Lys Gly Arg Tyr Asp
945                 950                 955                 960
Ala Cys Ser Gly Asp Ser Gly Gly Pro Leu Val Phe Ala Asp Asp Ser
                965                 970                 975
Arg Thr Glu Arg Arg Trp Val Leu Glu Gly Ile Val Ser Trp Gly Ser
            980                 985                 990
Pro Ser Gly Cys Gly Lys Ala Asn  Gln Tyr Gly Gly Phe  Thr Lys Val
        995                 1000                 1005
Asn Val  Phe Leu Ser Trp Ile  Arg Gln Phe Ile
    1010                 1015

<210>  5
<211>  1203
<212>  DNA
<213>  Tachypleus tridentatus

<220>
<223>  Tachypleus tridentatus factor B

<400>  5
atgacgtgga tatgtgtgat aacgttgttt gctctggctt ctgctacgtt gggtaacaaa      60
gttagtagag tgggggtcct cttccccaag acacggaacg acaatgagtg tacagcaaga     120
gggggattga aaggatcctg caaatccctc atagactgtc ctagtgtctt ggctacgttg     180
aaggacagtt ttcctgtcgt ttgctcttgg aatggtcgat ttcagcctat tgtctgctgt     240
cctgatgcaa tagcaccacc acctgtaacc acaacagctg taactgtaat atctacaaaa     300
gaaccaaagc ttccaagatt acatatatca ggttgtggaa aaagaaaagt caaaatagat     360
attacaactg ttggacgctc tggatcacca atacttcctc cgatatctac tcctcaaaat     420
tcaacaggtg ggagaggaat tattgctgga ggcgtagaag ccaaaattgg cgcgtggcct     480
tggatggcag ctgtttttgt gaaaaacttt ggcattggca gattccactg tgctggtagc     540
ataatcagta acaagtacat tttgtcagct gcccacgcct tccttatcgg aggtcgaaag     600
ttgaccccaa ctcgcttagc tgtccgtgtg ggaggccact acataaagag gggtcaagag     660
tatccagtga aagacgtgat tatccatcct cattatgtag aaaaggagaa ctacaatgat     720

atagccataa tcgagttaaa agaggaactg aactttacgg acttggtcaa tcctatatgt 780
ctccctgatc cagagacagt aacggatcca ttaaaagaca gaattgtgac tgcagcggga 840
tggggcgatc tggatttctc cggtccacgg agccaagttc tacgtgaggt aagcatccca 900
gttgttccag ttgataaatg tgatcaagcc tatgagaaac tcaacacccc ttcactaaaa 960
aatgggataa cgaataactt cctttgcgct ggattggaag aaggagggaa agacgcttgc 1020
caaggcgatt ctggtggacc gttgatgcta gtgaacaaca ctaggtggat agtagtagga 1080
gttgtgtcgt tcgggcacaa gtgtgccgag gaaggatatc ctggtgtgta ctcgcgcgta 1140
gcgagttacc tagactggat cgcgaaagtt acgaactcgt tagatcatgc cgtcactaac 1200
taa 1203

<210> 6
<211> 400
<212> PRT
<213> Tachypleus tridentatus

<220>
<223> Tachypleus tridentatus factor B

<400> 6
Met Thr Trp Ile Cys Val Ile Thr Leu Phe Ala Leu Ala Ser Ala Thr
1               5                   10                  15
Leu Gly Asn Lys Val Ser Arg Val Gly Val Leu Phe Pro Lys Thr Arg
            20                  25                  30
Asn Asp Asn Glu Cys Thr Ala Arg Gly Gly Leu Lys Gly Ser Cys Lys
        35                  40                  45
Ser Leu Ile Asp Cys Pro Ser Val Leu Ala Thr Leu Lys Asp Ser Phe
    50                  55                  60
Pro Val Val Cys Ser Trp Asn Gly Arg Phe Gln Pro Ile Val Cys Cys
65                  70                  75                  80
Pro Asp Ala Ile Ala Pro Pro Pro Val Thr Thr Thr Ala Val Thr Val
                85                  90                  95
Ile Ser Thr Lys Glu Pro Lys Leu Pro Arg Leu His Ile Ser Gly Cys
            100                 105                 110
Gly Lys Arg Lys Val Lys Ile Asp Ile Thr Thr Val Gly Arg Ser Gly
        115                 120                 125
Ser Pro Ile Leu Pro Pro Ile Ser Thr Pro Gln Asn Ser Thr Gly Gly
    130                 135                 140
Arg Gly Ile Ile Ala Gly Gly Val Glu Ala Lys Ile Gly Ala Trp Pro
145                 150                 155                 160
Trp Met Ala Ala Val Phe Val Lys Asn Phe Gly Ile Gly Arg Phe His
                165                 170                 175
Cys Ala Gly Ser Ile Ile Ser Asn Lys Tyr Ile Leu Ser Ala Ala His
            180                 185                 190
Ala Phe Leu Ile Gly Gly Arg Lys Leu Thr Pro Thr Arg Leu Ala Val
        195                 200                 205
Arg Val Gly Gly His Tyr Ile Lys Arg Gly Gln Glu Tyr Pro Val Lys
    210                 215                 220
Asp Val Ile Ile His Pro His Tyr Val Glu Lys Glu Asn Tyr Asn Asp
225                 230                 235                 240
Ile Ala Ile Ile Glu Leu Lys Glu Glu Leu Asn Phe Thr Asp Leu Val
                245                 250                 255
Asn Pro Ile Cys Leu Pro Asp Pro Glu Thr Val Thr Asp Pro Leu Lys
            260                 265                 270
Asp Arg Ile Val Thr Ala Ala Gly Trp Gly Asp Leu Asp Phe Ser Gly
        275                 280                 285
Pro Arg Ser Gln Val Leu Arg Glu Val Ser Ile Pro Val Val Pro Val
    290                 295                 300
Asp Lys Cys Asp Gln Ala Tyr Glu Lys Leu Asn Thr Pro Ser Leu Lys
305                 310                 315                 320
Asn Gly Ile Thr Asn Asn Phe Leu Cys Ala Gly Leu Glu Glu Gly Gly
                325                 330                 335
Lys Asp Ala Cys Gln Gly Asp Ser Gly Gly Pro Leu Met Leu Val Asn

                340                 345                 350
Asn Thr Arg Trp Ile Val Val Gly Val Val Ser Phe Gly His Lys Cys
        355                 360                 365
Ala Glu Glu Gly Tyr Pro Gly Val Tyr Ser Arg Val Ala Ser Tyr Leu
    370                 375                 380
Asp Trp Ile Ala Lys Val Thr Asn Ser Leu Asp His Ala Val Thr Asn
385                 390                 395                 400

<210>  7
<211>  1128
<212>  DNA
<213>  Tachypleus tridentatus

<220>
<223>  Tachypleus tridentatus pro-clotting enzyme

<400>  7
atgttggtga ataacgtgtt ttcactactg tgtttcccac tcttgatgtc tgtggttaga      60
tgcagtactc tcagcagaca gcgtagacag tttgttttcc ctgacgagga agaactttgc     120
tcaaaccgat ttactgaaga aggaacatgc aaaaatgtct tggattgtag aatactttta     180
caaaaaaatg attataattt actcaaagaa tcaatatgcg gctttgaagg cataacaccc     240
aaagtttgtt gtccgaaatc aagccatgta atttcaagta cacaggcacc tccagaaacc     300
actacgactg aacgcccacc aaaacagata ccacccaatc ttcctgaagt gtgtggaatt     360
cacaatacta caactaccag gattattgga ggtcgggaag cacctattgg agcctggccg     420
tggatgactg ctgtctacat aaaacaagga ggaatcagaa gtgttcagtg tggtggcgca     480
cttgtcacta acaggcacgt gattacagct tcgcactgtg ttgtaaacag tgcaggaaca     540
gatgtgatgc cagctgatgt attctcggtt cgtctgggtg aacacaattt atacagtacc     600
gatgacgatt cgaatccaat agattttgca gttacgtcgg tgaaacatca cgaacacttt     660
gtactcgcga cgtatttgaa tgacatcgca attctaacgt taaatgacac agttacgttt     720
acagacagaa ttcgacccat ttgtctacct tatcgtaagt tgagatacga tgatctagca     780
atgagaaaac cgtttatcac tggatgggga acaacagcat ttaacggccc atctagtgca     840
gtgttgagag aagtacagtt accaatatgg gaacacgagg cctgtagaca ggcctacgag     900
aaggatttaa atattacaaa cgtgtatatg tgtgctggct ttgcagatgg cgggaaggat     960
gcttgccagg gtgattctgg aggtccaatg atgttgcctg ttaaaaccgg agagttttat    1020
ctcattggaa ttgtgtcttt cggaaagaaa tgcgcattgc ctggatttcc tggggtttac    1080
acaaaagtga cagagttttt agattggatt gcagaacata tggtgtag                 1128

<210>  8
<211>  375
<212>  PRT
<213>  Tachypleus tridentatus

<220>
<223>  Tachypleus tridentatus pro-clotting enzyme

<400>  8
Met Leu Val Asn Asn Val Phe Ser Leu Leu Cys Phe Pro Leu Leu Met
1               5                   10                  15
Ser Val Val Arg Cys Ser Thr Leu Ser Arg Gln Arg Arg Gln Phe Val
            20                  25                  30
Phe Pro Asp Glu Glu Glu Leu Cys Ser Asn Arg Phe Thr Glu Glu Gly
        35                  40                  45
Thr Cys Lys Asn Val Leu Asp Cys Arg Ile Leu Leu Gln Lys Asn Asp
    50                  55                  60
Tyr Asn Leu Leu Lys Glu Ser Ile Cys Gly Phe Glu Gly Ile Thr Pro
65                  70                  75                  80
Lys Val Cys Cys Pro Lys Ser Ser His Val Ile Ser Ser Thr Gln Ala
                85                  90                  95
Pro Pro Glu Thr Thr Thr Thr Glu Arg Pro Pro Lys Gln Ile Pro Pro
            100                 105                 110
Asn Leu Pro Glu Val Cys Gly Ile His Asn Thr Thr Thr Thr Arg Ile

115                 120                 125
Ile Gly Gly Arg Glu Ala Pro Ile Gly Ala Trp Pro Trp Met Thr Ala
    130                 135                 140
Val Tyr Ile Lys Gln Gly Gly Ile Arg Ser Val Gln Cys Gly Gly Ala
145                 150                 155                 160
Leu Val Thr Asn Arg His Val Ile Thr Ala Ser His Cys Val Val Asn
                165                 170                 175
Ser Ala Gly Thr Asp Val Met Pro Ala Asp Val Phe Ser Val Arg Leu
            180                 185                 190
Gly Glu His Asn Leu Tyr Ser Thr Asp Asp Asp Ser Asn Pro Ile Asp
        195                 200                 205
Phe Ala Val Thr Ser Val Lys His His Glu His Phe Val Leu Ala Thr
    210                 215                 220
Tyr Leu Asn Asp Ile Ala Ile Leu Thr Leu Asn Asp Thr Val Thr Phe
225                 230                 235                 240
Thr Asp Arg Ile Arg Pro Ile Cys Leu Pro Tyr Arg Lys Leu Arg Tyr
                245                 250                 255
Asp Asp Leu Ala Met Arg Lys Pro Phe Ile Thr Gly Trp Gly Thr Thr
            260                 265                 270
Ala Phe Asn Gly Pro Ser Ser Ala Val Leu Arg Glu Val Gln Leu Pro
        275                 280                 285
Ile Trp Glu His Glu Ala Cys Arg Gln Ala Tyr Glu Lys Asp Leu Asn
    290                 295                 300
Ile Thr Asn Val Tyr Met Cys Ala Gly Phe Ala Asp Gly Gly Lys Asp
305                 310                 315                 320
Ala Cys Gln Gly Asp Ser Gly Gly Pro Met Met Leu Pro Val Lys Thr
                325                 330                 335
Gly Glu Phe Tyr Leu Ile Gly Ile Val Ser Phe Gly Lys Lys Cys Ala
            340                 345                 350
Leu Pro Gly Phe Pro Gly Val Tyr Thr Lys Val Thr Glu Phe Leu Asp
        355                 360                 365
Trp Ile Ala Glu His Met Val
    370                 375

<210>  9
<211>  1203
<212>  DNA
<213>  Tachypleus tridentatus

<220>
<223>  Tachypleus tridentatus factor B, codon optimized sequence

<400>  9
atgacctgga tctgcgtgat caccctgttc gctctggctt ccgctaccct gggcaacaag      60
gtgtcccgtg tgggtgtcct gttccccaag acccgtaacg acaacgagtg caccgctcgt     120
ggtggtctga agggctcctg caagtccctg atcgactgcc cctccgtgct ggctaccctg     180
aaggactcct tccccgtcgt gtgctcctgg aacggtcgtt tccagcccat cgtgtgctgc     240
cccgacgcta tcgctccccc ccctgtgacc accaccgctg tgaccgtgat ctccaccaag     300
gagcccaagc tgccccgtct gcacatctcc ggttgcggca agcgcaaggt caagatcgac     360
atcaccaccg tgggccgttc cggttccccc atcctgcccc ccatctccac cccccagaac     420
tccactggtg gtcgtggtat catcgctggc ggtgtcgagg ctaagatcgg tgcttggccc     480
tggatggctg ctgtgttcgt gaagaacttc ggtatcggtc gcttccactg cgctggttcc     540
atcatctcca acaagtacat cctgtccgct gctcacgctt tcctcatcgg tggtcgcaag     600
ctgaccccca cccgtctggc tgtgcgtgtg ggtggtcact acatcaagcg tggccaggag     660
taccccgtca aggacgtgat catccacccc cactacgtgg agaaggagaa ctacaacgac     720
atcgccatca tcgagctgaa ggaggagctg aacttcaccg acctggtcaa ccccatctgc     780
ctgcccgacc ccgagactgt gaccgaccct ctgaaggacc gtatcgtgac cgctgctggc     840
tggggcgacc tggacttctc cggtccccgt tcccaggtgc tgcgtgaggt gtccatcccc     900
gtggtgcccg tggacaagtg cgaccaggct tacgagaagc tgaacacccc ctccctgaag     960
aacggtatta ccaacaactt cctctgcgcc ggactcgagg agggtggcaa ggacgcttgc    1020
cagggcgact ccggtggtcc cctgatgctg gtcaacaaca cccgttggat cgtcgtgggt    1080
gtcgtgtcct tcggtcacaa gtgcgctgag gagggttacc ccggcgtcta ctcccgtgtg    1140

gcttcctacc tggactggat cgctaaggtc accaactccc tggaccacgc tgtcaccaac    1200
taa                                                                  1203

<210>  10
<211>  3063
<212>  DNA
<213>  Limulus polyphemus

<220>
<223>  Limulus polyphemus factor C

<400>  10
atggtactag cgtcgttctt ggtgtctggt ttagttctag ggctattagc ccaacaaatg      60
cacccagttc agtccagagg agtagatctg ggcttgtgtg atgacacgag gtttgagtgt     120
aagtgtggag atccaggata cgtgttcaac gtccccgcga agcaatgtac gtacttctat     180
cgatggaggc cttattgtaa accatgtgac aaactggagg ctaaagatgt gtgtcccaag     240
tacaaacgat gtcaagagtg tagggctggt ctcgacagtt gtgtgagttg tccacctaac     300
aaatatggaa cttggtgtag cggtgagtgt cagtgtaaga atgggggtat ttgtgatcag     360
aggacaggag cttgtacatg tcgtgacaga tatgaaggtg tgcattgtga aatccttcaa     420
ggttgtcctc ttcttcaatc ggatccccag gttcaggaag taaaaaatcc accaaatgat     480
ccacaaacta ttgactacag ctgttcacca ggcttcaagc ttaaaggcgt ggcacgtatc     540
acctgtcttc caaatggcca gtggagtagc tttccaccca aatgtattcg agaatgttcc     600
atggtttcat ctctagaaca tggcaaagta aactctccta gtgccgatct gatagaagga     660
gctactttaa ggttctcatg tgatagtccc tactacttga ttggtcaaga aacattaacc     720
tgccagggca acggtcagtg gagtgggcag ataccacagt gtcagaaatt ggtcttctgc     780
cctgaccttg accctgtaag ccatgctgaa caccaggtta aaattggcct agaacaaaaa     840
tatggtcaat ttcctcaagg cactgaagta acctatacgt gtactggtaa ttacttcttg     900
atgggtttgg acaccttaaa atgtaaccct gatgggtcct ggtcgggaac acagccgtcc     960
tgtgttaaag tggcagacag agaggtcaac tgtgacagta aagctgtgga cttcttggat    1020
gatgttggcg aacctgtcag gatccactgt cctgctggct gttccttaac tgctggtact    1080
gtatggggta cagccatata tcacgaactt tcctcagtat gtcgtgcagc tattcatgct    1140
ggcaaggttc caaactctgg aggtgcagtg catgtagtga acaacggtcc gtactcagac    1200
tttctggcta gtgatctgaa tgggataaaa tcagacgagt tgaagtctct tgctcagagt    1260
ttccgattcg attatgtcag ttcatcaaca gcagggagaa agtcaggatg tcctgatgga    1320
tggttcgaga ttgaggagaa ctgtgtgtac gttacatcga aacagagagc ctgggaaaga    1380
gctcaaggtg tatgtaccaa tatggccgct cgtcttgctg tgttagacaa agatgtaatt    1440
ccaagttcct tgactgagac tctacgaggg aaagggttag caacgacgtg gattggacta    1500
cacagattag atgctgataa tcactttatt tgggagctaa tggatcgcag tagtgttgct    1560
ttgaatgaca gcctaacatt ctgggctcct ggagaacctg ggaatgaaac taactgtgta    1620
tatctggata tccaagatca gctacagcca gtgtggaaaa ccaagtcttg ttttcaaccc    1680
tcaagttttg tttgtatgat ggatttgtca gacaagaaca aagccaaatg caaagaccct    1740
ggacctttgg aaaacggaca cgccaagctt catggtcaaa gtattgatgg attttatgct    1800
gggtcttctg taagatacag ctgcgaggtc ctccactacc tcagtggaac tgagacagta    1860
tcttgtacat caaatggcac gtggagtgcc cctaaacctc gatgtattaa agtcatcacc    1920
tgccaaaccc ctcctgtacc atcctatggt tctgtggaca tcaaaccccc aagtagaaca    1980
aactcaatca gtcgtgttgg gtcgccattc ttgaggttgc cacggttacc cctcccttta    2040
gccagagcag ccggacctcc tccaaaacct agatccgcac caccctctac tgtggacctg    2100
tcttccaagg tcaaactgcc tgaaggtcat taccgggtgg ggtctcaagc catttacacg    2160
tgcgagtcaa gatactacga actgcttgga tctcaaggta gaagatgcga ctctaatgga    2220
aagtggagtg gtcgaccagc aagctgtata ccagtttgtg gacggtcaga ctctccccgt    2280
tctcctttca tcgtcaatgg aaattccacc gaaataggtc agtggccgtg gcaggcagga    2340
atctccagat ggcttgcaga tcataatatg tggtttcttc agtgtggagg agctctactg    2400
aatgagaaat ggatcattac tgcagcccac tgtgtcacct actctgctac tgccgagatc    2460
attgacccaa gtcagtttaa attctacctg ggcaaatact atcgagatga cagtaaggat    2520
gatgactacg tacaagtaag agaggctatc gagatccatg tgaatcctaa ctacgatcct    2580
ggaaatctca actttgacat agccctgatt caactgaaga cttctgttgc tctgaccaca    2640
cgagtgcaac caatatgtct gcctactgat ctcactacaa gagaaaacct gaaagaggga    2700
gcgttagcgg tggtgacagg atgggggttg aatgaaaaca acacatattc agagatgatt    2760
cagcaagccg ttctgcctgt tgttgcagca agcacctgtg aacaaggata tcaggactcg    2820
ggcttgccac tgacagtgac agagaacatg ttctgtgcag gttacaagca ggggcgctat    2880
gatgcctgca gtggagacag tggaggacca ttagtgtttg ctgatgattc ccgcaccgat    2940
aggcggtggg tcctggaagg gatcgtcagc tggggcagcc ccaatggatg tggcaagtct    3000

aaccagtatg ggggcttcac taaagttaac gtttttctat cgtggattcg gcagttcatt 3060
tga 3063

<210> 11
<211> 3063
<212> DNA
<213> Limulus polyphemus

<220>
<223> Limulus polyphemus factor C, codon optimized sequence

<400> 11
atggtgctgg ccagcttcct ggtgtctggg ctggtgctgg ggcttctggc ccagcagatg 60
caccctgttc aatccagagg ggttgacctt ggcctgtgcg atgatacccg ctttgaatgt 120
aagtgcgggg atcccgggta cgtctttaac gtgcccgcta agcaatgcac atacttctac 180
cggtggcggc catattgcaa accttgcgac aagcttgagg caaaagacgt atgtcccaag 240
tataaacgat gtcaggagtg tcgggctggc ctggatagct gtgtgtcctg cccacctaac 300
aagtacggaa cctggtgttc aggcgagtgc cagtgcaaga acggtggaat ttgtgatcag 360
cgtacaggtg cctgtacatg tcgtgacaga tacgaaggag tgcactgcga gattctgcag 420
ggctgcccct tgctccagtc tgacccacag gtgcaggaag tcaagaatcc tcccaacgat 480
cctcagacga ttgactattc ctgctcaccc ggttttaagc tcaagggcgt ggcccgaatt 540
acttgtctcc ccaatggtca gtggtccagc tttcctccca agtgcattag agaatgtagc 600
atggtgtcca gtttggagca tggtaaggtc aacagtcctt cagctgacct cattgagggc 660
gcaacactcc ggttttcctg tgactctccc tattacttga ttggacagga aaccttgact 720
tgtcagggta atggccaatg gagtggtcag atcccacagt gccagaaact ggtcttttgc 780
cccgacctgg atcccgtcag ccatgccgaa caccaagtga aaatcgggct cgagcaaaag 840
tatgggcagt ttccacaggg caccgaggtg acctacacgt gtactggcaa ttactttctg 900
atgggactgg ataccctcaa atgcaatccc gacggaagtt ggagtggtac acagccatct 960
tgtgttaaag tcgcagatag ggaagtaaat tgcgattcca aggcagtaga ctttctggat 1020
gacgttgggg aacctgttcg catccattgt ccagctggtt gttccttgac tgctggtacc 1080
gtgtggggca cagctatcta tcacgagttg tcctcagtct gtagagctgc catacacgct 1140
ggcaaggtcc ctaactctgg aggcgccgtc catgtcgtga ataacggtcc atactctgac 1200
tttctggcct ccgaccttaa cggaatcaag tcagacgagc ttaaatcctt ggcacagtct 1260
ttccgcttcg actatgtgag ctcttctacc gcaggacgta agagcggctg tcccgacggc 1320
tggttcgaga tcgaagagaa ctgcgtgtac gtgacttcta agcagagggc ttgggagcga 1380
gcacagggag tatgcacaaa catggccgcc aggctcgctg tgctggataa ggacgtcatc 1440
cccagtagcc tgacagaaac actgagaggc aaagggctcg ccacaacttg gattggactg 1500
catcgcttgg acgcagataa ccactttatc tgggagctta tggatcgcag ctccgtggct 1560
ttgaacgatt ctttgacctt ttgggcaccc ggggagccag gcaatgagac taattgcgtc 1620
tacctggaca tacaggatca gctgcaaccc gtgtggaaaa cgaaatcttg cttccaaccc 1680
tcctcctttg tgtgcatgat ggatctgagc gacaagaaca aagccaagtg caaagatccc 1740
ggacctctgg agaatggcca tgcaaagctc catggacagt caattgatgg gttctatgcc 1800
ggcagttccg tccgttactc ctgcgaagtg ctccactacc tgagtgggac cgaaactgtg 1860
tcatgcacat ccaatggcac ctggagtgct cccaaaccta gatgtattaa ggtcatcact 1920
tgccaaactc cacccgtgcc cagctacgga tctgttgaca tcaagcctcc tagtaggaca 1980
aacagcataa gccgagtggg atcacccttc ctcagactgc ccagacttcc tctgcctctg 2040
gcccgcgctg ccggacctcc accaaagcca cgtagtgcac ctccctctac cgttgacctg 2100
tcctcaaaag tgaagctgcc agaaggccat taccgcgtcg ggtcacaagc catctatacg 2160
tgtgaaagtc gctactatga gctgctgggc agccaaggca ggcggtgtga ctccaatgga 2220
aagtggagcg gacgaccagc ctcatgcatt ccagtctgtg gcaggagcga ttccccacgg 2280
agtccattca tcgtcaatgg caattctaca gagattggtc agtggccttg gcaggctggt 2340
atctctcggt ggttggccga ccacaacatg tggtttctcc agtgtggcgg cgctctcctg 2400
aacgagaaat ggattatcac agcagctcac tgcgtaacct atagcgcaac cgccgagata 2460
atcgatccat cacagttcaa gttttacctg ggaaagtact acaggacgga tagtaaagat 2520
gacgactacg tgcaagtgag agaggccatc gagatccacg ttaaccccaa ctatgatcct 2580
gggaatctga atttcgatat tgccctgatc cagcttaaaa ccagcgttgc tctgactaca 2640
cgagtgcagc ccatatgtct cccaactgac ctgaccacca gggagaacct gaaggaaggg 2700
gcccttgctg ttgttaccgg ttggggtctg aatgagaata acacttactc tgaaatgatc 2760
caacaggcag tgctgcctgt ggtagctgcc tctacgtgcg aacagggata tcaggactca 2820
ggacttcctc tgacagtaac cgaaaacatg ttctgcgccg ggtataaaca gggccgctat 2880
gacgcctgtt ccggggattc cggcgggcct ctggttttcg ccgatgacag ccggactgat 2940
cggcgatggg tgcttgaggg tatcgtatcc tggggctctc ccaacggatg cggtaagagc 3000

aatcagtatg gtggcttcac caaagtgaac gttttcctgt cctggataag gcagttcatc 3060
tga 3063

<210> 12
<211> 1020
<212> PRT
<213> Limulus polyphemus

<220>
<223> Limulus polyphemus factor C

<400> 12
Met Val Leu Ala Ser Phe Leu Val Ser Gly Leu Val Leu Gly Leu Leu
1               5                   10                  15
Ala Gln Gln Met His Pro Val Gln Ser Arg Gly Val Asp Leu Gly Leu
            20                  25                  30
Cys Asp Asp Thr Arg Phe Glu Cys Lys Cys Gly Asp Pro Gly Tyr Val
        35                  40                  45
Phe Asn Val Pro Ala Lys Gln Cys Thr Tyr Phe Tyr Arg Trp Arg Pro
    50                  55                  60
Tyr Cys Lys Pro Cys Asp Lys Leu Glu Ala Lys Asp Val Cys Pro Lys
65                  70                  75                  80
Tyr Lys Arg Cys Gln Glu Cys Arg Ala Gly Leu Asp Ser Cys Val Ser
                85                  90                  95
Cys Pro Pro Asn Lys Tyr Gly Thr Trp Cys Ser Gly Glu Cys Gln Cys
            100                 105                 110
Lys Asn Gly Gly Ile Cys Asp Gln Arg Thr Gly Ala Cys Thr Cys Arg
        115                 120                 125
Asp Arg Tyr Glu Gly Val His Cys Glu Ile Leu Gln Gly Cys Pro Leu
    130                 135                 140
Leu Gln Ser Asp Pro Gln Val Gln Glu Val Lys Asn Pro Pro Asn Asp
145                 150                 155                 160
Pro Gln Thr Ile Asp Tyr Ser Cys Ser Pro Gly Phe Lys Leu Lys Gly
                165                 170                 175
Val Ala Arg Ile Thr Cys Leu Pro Asn Gly Gln Trp Ser Ser Phe Pro
            180                 185                 190
Pro Lys Cys Ile Arg Glu Cys Ser Met Val Ser Ser Leu Glu His Gly
        195                 200                 205
Lys Val Asn Ser Pro Ser Ala Asp Leu Ile Glu Gly Ala Thr Leu Arg
    210                 215                 220
Phe Ser Cys Asp Ser Pro Tyr Tyr Leu Ile Gly Gln Glu Thr Leu Thr
225                 230                 235                 240
Cys Gln Gly Asn Gly Gln Trp Ser Gly Gln Ile Pro Gln Cys Gln Lys
                245                 250                 255
Leu Val Phe Cys Pro Asp Leu Asp Pro Val Ser His Ala Glu His Gln
            260                 265                 270
Val Lys Ile Gly Leu Glu Gln Lys Tyr Gly Gln Phe Pro Gln Gly Thr
        275                 280                 285
Glu Val Thr Tyr Thr Cys Thr Gly Asn Tyr Phe Leu Met Gly Leu Asp
    290                 295                 300
Thr Leu Lys Cys Asn Pro Asp Gly Ser Trp Ser Gly Thr Gln Pro Ser
305                 310                 315                 320
Cys Val Lys Val Ala Asp Arg Glu Val Asn Cys Asp Ser Lys Ala Val
                325                 330                 335
Asp Phe Leu Asp Asp Val Gly Glu Pro Val Arg Ile His Cys Pro Ala
            340                 345                 350
Gly Cys Ser Leu Thr Ala Gly Thr Val Trp Gly Thr Ala Ile Tyr His
        355                 360                 365
Glu Leu Ser Ser Val Cys Arg Ala Ala Ile His Ala Gly Lys Val Pro
    370                 375                 380
Asn Ser Gly Gly Ala Val His Val Val Asn Asn Gly Pro Tyr Ser Asp
385                 390                 395                 400

Phe Leu Ala Ser Asp Leu Asn Gly Ile Lys Ser Asp Glu Leu Lys Ser
                405                 410                 415
Leu Ala Gln Ser Phe Arg Phe Asp Tyr Val Ser Ser Ser Thr Ala Gly
            420                 425                 430
Arg Lys Ser Gly Cys Pro Asp Gly Trp Phe Glu Ile Glu Glu Asn Cys
        435                 440                 445
Val Tyr Val Thr Ser Lys Gln Arg Ala Trp Glu Arg Ala Gln Gly Val
    450                 455                 460
Cys Thr Asn Met Ala Ala Arg Leu Ala Val Leu Asp Lys Asp Val Ile
465                 470                 475                 480
Pro Ser Ser Leu Thr Glu Thr Leu Arg Gly Lys Gly Leu Ala Thr Thr
                485                 490                 495
Trp Ile Gly Leu His Arg Leu Asp Ala Asp Asn His Phe Ile Trp Glu
            500                 505                 510
Leu Met Asp Arg Ser Ser Val Ala Leu Asn Asp Ser Leu Thr Phe Trp
        515                 520                 525
Ala Pro Gly Glu Pro Gly Asn Glu Thr Asn Cys Val Tyr Leu Asp Ile
    530                 535                 540
Gln Asp Gln Leu Gln Pro Val Trp Lys Thr Lys Ser Cys Phe Gln Pro
545                 550                 555                 560
Ser Ser Phe Val Cys Met Met Asp Leu Ser Asp Lys Asn Lys Ala Lys
                565                 570                 575
Cys Lys Asp Pro Gly Pro Leu Glu Asn Gly His Ala Lys Leu His Gly
            580                 585                 590
Gln Ser Ile Asp Gly Phe Tyr Ala Gly Ser Ser Val Arg Tyr Ser Cys
        595                 600                 605
Glu Val Leu His Tyr Leu Ser Gly Thr Glu Thr Val Ser Cys Thr Ser
    610                 615                 620
Asn Gly Thr Trp Ser Ala Pro Lys Pro Arg Cys Ile Lys Val Ile Thr
625                 630                 635                 640
Cys Gln Thr Pro Pro Val Pro Ser Tyr Gly Ser Val Asp Ile Lys Pro
                645                 650                 655
Pro Ser Arg Thr Asn Ser Ile Ser Arg Val Gly Ser Pro Phe Leu Arg
            660                 665                 670
Leu Pro Arg Leu Pro Leu Pro Leu Ala Arg Ala Ala Gly Pro Pro Pro
        675                 680                 685
Lys Pro Arg Ser Ala Pro Pro Ser Thr Val Asp Leu Ser Ser Lys Val
    690                 695                 700
Lys Leu Pro Glu Gly His Tyr Arg Val Gly Ser Gln Ala Ile Tyr Thr
705                 710                 715                 720
Cys Glu Ser Arg Tyr Tyr Glu Leu Leu Gly Ser Gln Gly Arg Arg Cys
                725                 730                 735
Asp Ser Asn Gly Lys Trp Ser Gly Arg Pro Ala Ser Cys Ile Pro Val
            740                 745                 750
Cys Gly Arg Ser Asp Ser Pro Arg Ser Pro Phe Ile Val Asn Gly Asn
        755                 760                 765
Ser Thr Glu Ile Gly Gln Trp Pro Trp Gln Ala Gly Ile Ser Arg Trp
    770                 775                 780
Leu Ala Asp His Asn Met Trp Phe Leu Gln Cys Gly Gly Ala Leu Leu
785                 790                 795                 800
Asn Glu Lys Trp Ile Ile Thr Ala Ala His Cys Val Thr Tyr Ser Ala
                805                 810                 815
Thr Ala Glu Ile Ile Asp Pro Ser Gln Phe Lys Phe Tyr Leu Gly Lys
            820                 825                 830
Tyr Tyr Arg Asp Asp Ser Lys Asp Asp Asp Tyr Val Gln Val Arg Glu
        835                 840                 845
Ala Ile Glu Ile His Val Asn Pro Asn Tyr Asp Pro Gly Asn Leu Asn
    850                 855                 860
Phe Asp Ile Ala Leu Ile Gln Leu Lys Thr Ser Val Ala Leu Thr Thr
865                 870                 875                 880
Arg Val Gln Pro Ile Cys Leu Pro Thr Asp Leu Thr Thr Arg Glu Asn
                885                 890                 895
Leu Lys Glu Gly Ala Leu Ala Val Val Thr Gly Trp Gly Leu Asn Glu

            900                 905                 910
Asn Asn Thr Tyr Ser Glu Met Ile Gln Gln Ala Val Leu Pro Val Val
        915                 920                 925
Ala Ala Ser Thr Cys Glu Gln Gly Tyr Gln Asp Ser Gly Leu Pro Leu
    930                 935                 940
Thr Val Thr Glu Asn Met Phe Cys Ala Gly Tyr Lys Gln Gly Arg Tyr
945                 950                 955                 960
Asp Ala Cys Ser Gly Asp Ser Gly Gly Pro Leu Val Phe Ala Asp Asp
                965                 970                 975
Ser Arg Thr Asp Arg Arg Trp Val Leu Glu Gly Ile Val Ser Trp Gly
            980                 985                 990
Ser Pro Asn Gly Cys Gly Lys Ser Asn Gln Tyr Gly Gly Phe Thr Lys
        995                 1000                1005
Val Asn Val Phe Leu Ser Trp Ile Arg Gln Phe Ile
    1010                1015                1020

## Description

### Technical Field

The present invention relates to a method for enhancing the sensitivity of an endotoxin measuring reagent to the endotoxin of Helicobacter pylori.

### Background Art

It is known that an extract of amoebocyte present in blood of a horseshoe crab (amoebocyte lysate) coagulates when it contacts with an endotoxin. By taking advantage of this property, the amoebocyte lysate is widely used, as a reagent for detecting an endotoxin at high sensitivity, for quality management of a pharmaceutical product or the like.

The reagent is referred to as a "lysate reagent". Furthermore, as the property of causing coagulation by an endotoxin is initially found in Limulus polyphemus, there are also cases in which the reagent is referred to as a "Limulus reagent" or a "Limulus amoebocyte lysate (LAL) reagent".

For the protection of a horseshoe crab, there is an idea of producing the reagent by artificially produced proteins in an amoebocyte lysate based on recombination techniques (Patent Literatures 1 and 2). In fact, by using recombinant proteins, products like PyroGene (registered trademark) (LONZA JAPAN), EndoZyme (registered trademark) II (bioMérieux Japan Ltd.), and PyroSmart (registered trademark) (SEIKAGAKU CORPORATION) have been launched onto the market.

From the results of Ministry of Health, Labor, and Welfare Grants (Non Patent Literature 1) by Tanamoto et al. and reports by Loverock et al. (Non Patent Literature 2), Grallert et al. (Non Patent Literature 3), and Bolden et al. (Non Patent Literature 4), it was considered that the performance of the "recombinant reagent", which is an endotoxin measuring reagent using recombinant proteins, is equivalent to the lysate reagent.

### Citation List

### Patent Literature

Patent Literature 1: WO 2012/118226 A
Patent Literature 2: WO 2014/092079 A

### Non Patent Literature

Non Patent Literature 1: Document Number 201427027B of the results of Ministry of Health, Labor, and Welfare Grants "Research on Introduction of Advanced Test Method for Securing Microbiological Quality of Pharmaceutical Product"
Non Patent Literature 2: Loverock, B et al., "A Recombinant Factor C Procedure for the Detection of Gram-Negative Bacteria Endotoxina", Pharmacopeial Forum 36, 321-29, (2010 )
Non Patent Literature 3: Grallert, H et al., "EndoLISA: a novel and reliable method for endotoxin detection", Nature Methods, October (2011)
Non Patent Literature 4: Bolden, J. et al., "Application of Recombinant Factor C Reagent for the Detection of Bacteria Endotoxins in Pharmaceutical Products", PDA J Pharm Sci Technol., 2017, Sep-Oct, 71(5), 405-412 (2017)

### Summary of Invention

However, as a result of measuring the relative potency of the endotoxin of Helicobacter pylori in comparison with that of Reference Standard Endotoxin by using a current recombinant reagent, the inventors of the present invention found that the relative potency is significantly lower than that measured by using a lysate reagent. Accordingly, an object of the present invention is to provide a method for enhancing the sensitivity of an endotoxin measuring reagent using a recombinant protein to the endotoxin of Helicobacter pylori.

As a result of carrying out intensive studies to solve the above problems, the inventors of the present invention found that, by increasing a content of a recombinant protein of factor C in an endotoxin measuring reagent employing a recombinant protein at the time of endotoxin measurement (namely, co-existing with the endotoxin of Helicobacter pylori), the sensitivity to the endotoxin of Helicobacter pylori can be enhanced. Based on these findings, the inventors of the present invention completed the present invention.

Namely, the present invention relates to the followings.
[1] A method for enhancing a sensitivity of an endotoxin measuring reagent to the endotoxin of Helicobacter pylori (endotoxin derived from Helicobacter pylori),
   the endotoxin measuring reagent containing a recombinant protein of horseshoe crab factor C (factor C derived from a horseshoe crab),
   the method including increasing a content of the recombinant protein of factor C at the time of endotoxin measurement.
[2] The method described in [1], in which the endotoxin measuring reagent contains a recombinant protein of horseshoe crab factor B (factor B derived from a horseshoe crab).
[3] The method described in [1] or [2], in which the endotoxin measuring reagent contains a recombinant protein of horseshoe crab pro-clotting enzyme (pro-clotting enzyme derived from a horseshoe crab).
[4] The method described in any one of [1] to [3], in which the endotoxin measuring reagent contains a compound for detection.
[5] An endotoxin measuring method in which sensitivity of an endotoxin measuring reagent to the endotoxin of Helicobacter pylori is enhanced, the method including:
   preparing an endotoxin measurement sample containing the endotoxin measuring reagent and an analyte,
   in which the endotoxin measuring reagent contains a recombinant protein of horseshoe crab factor C, and
   the sensitivity is enhanced by increasing a concentration of the recombinant protein of factor C in the endotoxin measurement sample.
[6] The method described in [5], in which the endotoxin measuring reagent contains a recombinant protein of horseshoe crab factor B.
[7] The method described in [5] or [6], in which the endotoxin measuring reagent contains a recombinant protein of horseshoe crab pro-clotting enzyme.
[8] The method described in any one of [5] to [7], in which the endotoxin measuring reagent contains a compound for detection.
[9] An endotoxin measuring reagent having enhanced sensitivity to the endotoxin of Helicobacter pylori.
[10] A method for producing an endotoxin measuring reagent having enhanced sensitivity to the endotoxin of Helicobacter pylori,
   the endotoxin measuring reagent containing a recombinant protein of horseshoe crab factor C,
   the method including increasing a content of the recombinant protein of factor C in the endotoxin measuring reagent.
[11] An endotoxin measuring reagent containing a horseshoe crab factor C, in which the horseshoe crab factor C is a recombinant protein, and relative potency of the endotoxin of Helicobacter pylori is 0.1 times or more relative potency measured by using a lysate reagent.
[12] The endotoxin measuring reagent described in [11], in which the endotoxin measuring reagent contains a recombinant protein of horseshoe crab factor B.
[13] The endotoxin measuring reagent described in [11] or [12], in which the endotoxin measuring reagent contains a recombinant protein of horseshoe crab pro-clotting enzyme.
[14] The endotoxin measuring reagent described in any one of [11] to [13], in which the endotoxin measuring reagent contains a compound for detection.
[15] An endotoxin measuring reagent containing a horseshoe crab factor C, in which the horseshoe crab factor C is a recombinant protein, and relative potency of the endotoxin of Helicobacter pylori is 200 (EU/µg) or more.
[16] The endotoxin measuring reagent described in [15], in which the endotoxin measuring reagent contains a recombinant protein of horseshoe crab factor B.
[17] The endotoxin measuring reagent described in [15] or [16], in which the endotoxin measuring reagent contains a recombinant protein of horseshoe crab pro-clotting enzyme.
[18] The endotoxin measuring reagent described in any one of [15] to [17], in which the endotoxin measuring reagent contains a compound for detection. One aspect of the compound for detection is a compound for detection represented by general formula Y-X-Z, in which in the general formula, Y is a hydrogen atom or a protecting group, X is a peptide containing the amino acid sequence, which is a substrate of the recombinant protein of pro-clotting enzyme, and Z is a labeling material which becomes optically detectable when being released from X. Another aspect of the compound for detection is a compound for detection represented by general formula Y-X-Z, in which in the general formula, Y is a hydrogen atom or a protecting group, X is a peptide containing the amino acid sequence, which is a substrate of the recombinant protein of factor B, and Z becomes a labeling material which is optically detectable when being released from X. Still another aspect of the compound for detection is a compound for detection represented by general formula Y-X-Z, in which in the general formula, Y is a hydrogen atom or a protecting group, X is a peptide containing the amino acid sequence, which is a substrate of the factor C, and Z is a labeling material which becomes optically detectable when being released from X.
[19] A method for measuring endotoxin in an analyte by using the endotoxin measuring reagent described in any one of [11] to [18].

### Brief Description of Drawings

FIG. 1 is a diagram showing the relationship between the concentration of a recombinant factor C in a reaction solution and the relative potency of endotoxin of Helicobacter pylori CA2.
FIG. 2 is a diagram showing the relationship between the concentration of a recombinant factor B in a reaction solution and the relative potency of the endotoxin of Helicobacter pylori CA2.
FIG. 3 is a diagram showing the relationship between the concentration of a recombinant pro-clotting enzyme in a reaction solution and the relative potency of endotoxin of Helicobacter pylori CA2.

### Description of Embodiments

Abbreviations used in the present specification and meanings of the abbreviations are described.

Factor C (FC)
Recombinant factor C (rFC)
Factor B (FB)
Recombinant factor B (rFB)
Pro-clotting enzyme (coagulation enzyme precursor: PCE)
Recombinant pro-clotting enzyme (rPCE)
Clotting enzyme (coagulation enzyme)

According to the present invention, a method for enhancing the sensitivity of an endotoxin measuring reagent employing a recombinant protein to the endotoxin of Helicobacter pylori can be provided.

In the present specification, the factor C, the factor B, and the pro-clotting enzyme may be also referred to as, either individually or together, a "Limulus factor".

The present invention is described hereinbelow.

### <Method for enhancing sensitivity of endotoxin measuring reagent>

One aspect of the present invention relates to a method for enhancing the sensitivity of an endotoxin measuring reagent to the endotoxin of Helicobacter pylori, in which the endotoxin measuring reagent contains a recombinant protein of factor C of horseshoe crab and the method includes increasing a content of the recombinant protein of factor C at the time of endotoxin measurement (hereinbelow, the method may be also referred to as an "enhancing method of the present invention"). According to an embodiment of the present invention, the content of the recombinant protein of factor C at the time of endotoxin measurement is increased to an amount that is sufficient for enhancing the sensitivity.

Another aspect of the present invention relates to a method for improving the reactivity of an endotoxin measuring reagent to the endotoxin of Helicobacter pylori, in which the endotoxin measuring reagent contains a recombinant protein of horseshoe crab factor C, and the method includes increasing a content of the recombinant protein of factor C at the time of endotoxin measurement. Further aspect of the present invention relates to a method for imparting the reactivity of a recombinant protein of factor C of horseshoe crab to the endotoxin of Helicobacter pylori, the method including increasing an amount of the recombinant protein of factor C to co-exist with the endotoxin. In an embodiment of the present invention, the reactivity is expressed as relative potency of 200 EU/µg or higher (for example, 250 EU/µg or higher or 300 EU/µg or higher).

As described in the above, the inventors of the present invention recognized a problem that the sensitivity of a current endotoxin measuring reagent employing a recombinant protein to the endotoxin of Helicobacter pylori is significantly lower than that of a lysate reagent.

According to the enhancing method of the present invention, for measuring an endotoxin in which a recombinant reagent as an endotoxin measuring reagent employing a recombinant protein is used, by increasing the content of the recombinant protein of factor C at the time of endotoxin measurement, it is made possible to enhance the sensitivity of an endotoxin measuring reagent to the endotoxin of Helicobacter pylori. Namely, the relative potency of the endotoxin of Helicobacter pylori to Reference Standard Endotoxin can be enhanced. More specifically, by increasing the concentration of a recombinant protein of factor C in an endotoxin measurement sample which contains an endotoxin measuring reagent and an analyte, the sensitivity of an endotoxin measuring reagent to the endotoxin of Helicobacter pylori can be enhanced. Herein, as described in Examples described below, the inventors of the present invention recognized that, even when the concentration of a Limulus factor other than the recombinant protein of factor C is increased in an endotoxin measurement sample, the relative potency of the endotoxin of Helicobacter pylori with reference to that of Reference Standard Endotoxin cannot be increased or a higher background is caused so that the sensitivity of an endotoxin measuring reagent to the endotoxin of Helicobacter pylori cannot be enhanced.

According to the enhancing method of the present invention, the content of the recombinant protein of factor C that is sufficient for enhancing the sensitivity of an endotoxin measuring reagent at the time of endotoxin measurement can be varied depending on desired relative potency, measurement system, hosts of the recombinant protein of factor C, or the like, and, although not limited, the content of the recombinant protein of factor C can be increased such that the relative potency of the endotoxin of Helicobacter pylori relative to that of Reference Standard Endotoxin, which is measured by using an endotoxin measuring reagent employing a recombinant protein, is 0.1 times or more, 0.2 times or more, 0.3 times or more, 0.4 times or more, 0.5 times or more, 0.6 times or more, 0.7 times or more, 0.8 times or more, 0.9 times or more, or 1.0 times or more the relative potency measured by using a lysate reagent.

Herein, as for the lysate reagent, a hemocyte extract prepared from lymph fluid of horseshoe crab blood by a common method (see, J. Biochem., 80, 1011-1021 (1976), for example) can be mentioned. Furthermore, it is also possible to use the extract added with, if necessary, a divalent metal salt effective for factor C activation, a substrate of a clotting enzyme (for example, synthetic substrate described below), a pH adjusting reagent, and the like. Furthermore, as for the lysate reagent, commercially available products can be also used. The lysate reagent is not limited, and examples thereof include Endospecy (registered trademark) ES-50M (SEIKAGAKU CORPORATION).

Furthermore, measurement of the relative potency of the endotoxin of Helicobacter pylori by using an endotoxin measuring reagent applying a recombinant protein can be achieved by referring Reference Standard Endotoxin, and, for example, the measurement can be carried out according to the method described in Examples using a compound for detection. The measurement method may be either end-point measurement or kinetics measurement.

Specifically, as for measurement of the relative potency, for example, (i) absorbance of an endotoxin measurement sample is measured and an absorbance change ratio per unit time (mAbs/min) is calculated. (ii) Subsequently, from a calibration curve which has been established by using Reference Standard Endotoxin, the endotoxin activity (EU/mL) of an endotoxin measurement sample is calculated, and (iii) by dividing this value by the concentration (µg/mL) of endotoxin contained in an endotoxin measurement sample, the relative potency (EU/µg) of the endotoxin is calculated. The method using a change in absorbance over time is particularly suitable for measurement of the relative potency of endotoxin in a three factor system (a reaction system including a factor C, a factor B, and a pro-clotting enzyme). In an embodiment, the measurement of the relative potency of the endotoxin of Helicobacter pylori is carried out in a three factor system by the method using a change in absorbance over time.

The measurement of the relative potency may be carried out by a method based on fluorescence intensity measurement. More specifically, (i) a change in relative fluorescence intensity of an endotoxin measurement sample during a cascade reaction is measured by using an endotoxin measuring reagent, (ii) an endotoxin activity (EU/mL) of the endotoxin measurement sample is calculated from a calibration curve which has been established by using Reference Standard Endotoxin, and (iii) this value is divided by the concentration (µg/mL) of endotoxin contained in the endotoxin measurement sample to calculate the relative potency (EU/µg) of endotoxin. A method using a change in fluorescence intensity over time is particularly suitable for measurement of the relative potency in a one factor system (a reaction system including a factor C and not including a factor B or a pro-clotting enzyme) and measurement of the relative potency in a two factor system (a reaction system including a factor C and a factor B and not including a pro-clotting enzyme). In an embodiment, the measurement of the relative potency of the endotoxin of Helicobacter pylori is carried out in a one factor system or a two factor system by the method using a change in fluorescence intensity over time.

Herein, the amount of the endotoxin of Helicobacter pylori used in measurement of the relative potency is not particularly limited as long as it is an amount with which the endotoxin activity (EU/mL) of an endotoxin measurement sample (namely, a sample containing the endotoxin of Helicobacter pylori) can be calculated from a calibration curve which has been established by using Reference Standard Endotoxin, and the amount thereof can be suitably set depending on various conditions such as the type of a method of measuring relative potency. The amount of the endotoxin of Helicobacter pylori used in measurement of the relative potency may be, in terms of concentration in a measurement system, for example, 0.25 to 25 ng/mL, and may be specifically 0.25 ng/mL, 5 ng/mL, or 25 ng/mL. The amount of the endotoxin of Helicobacter pylori used in measurement of the relative potency may be, in terms of concentration in a measurement system, 0.25 to 25 ng/mL, and may be specifically 0.25 ng/mL, 5 ng/mL, or 25 ng/mL, particularly in the case of performing measurement of relative potency in a one factor system. In addition, the amount of the endotoxin of Helicobacter pylori used in measurement of the relative potency may be, in terms of concentration in a measurement system, 0.25 ng/mL particularly in the case of performing measurement of relative potency in a two factor system or a three factor system.

Herein, examples of Reference Standard of Endotoxin include a Reference Standard Endotoxin Japanese Pharmacopoeia, Reference Standard Endotoxin of US Pharmacopoeia, and Endotoxin Standard of European Pharmacopoeia.

In a case in which a commercially available recombinant reagent A that is used in Examples described below is used, for example, the relative potency of the endotoxin of Helicobacter pylori is 147.3 EU/µg (Table 1). On the other hand, in a case in which a commercially available lysate reagent A or lysate reagent B is used, the relative potency of endotoxin of Helicobacter pylori is about 2,000 EU/µg. In a case in which an endotoxin is measured based on a three factor system using a recombinant protein of factor C which has been expressed in mammalian cells, if the content of the recombinant protein of factor C at the time of endotoxin measurement (namely, in the measurement sample at the time of endotoxin measurement) is set at 162 ng/mL or more, 0.25 ng/ml of endotoxin of Helicobacter pylori becomes possible to achieve the relative potency of 200 EU/µg or higher, which is 0.1 times or more than the relative potency measured by using a lysate reagent (Table 2). In this case, by the increased content of the recombinant protein of factor C in the measurement sample to 162 ng/mL or more at the time of endotoxin measurement, the sensitivity of an endotoxin measuring reagent to the endotoxin of Helicobacter pylori can be enhanced. Furthermore, in Table 1, the content of the recombinant protein of factor C in a commercially available recombinant reagent A at the time of endotoxin measurement is less than 162 ng/mL.

The content of the recombinant protein of factor C in the measurement sample at the time of endotoxin measurement can be varied depending on desired relative potency, measurement system, hosts of the recombinant protein of factor C, or the like. In a case in which the measurement system is based on a three factor system, although it is not limited, the content of the recombinant protein of factor C in the measurement sample at the time of endotoxin measurement can be set at 140 ng/mL or more, 162 ng/mL or more, 180 ng/mL or more, 200 ng/mL or more, 250 ng/mL or more, 300 ng/mL or more, 400 ng/mL or more, 500 ng/mL or more, 600 ng/mL or more, or 700 ng/mL or more. In a case in which the measurement system is in a two factor system, although it is not limited, the content of the recombinant protein of factor C in the measurement sample at the time of endotoxin measurement can be set at 235 ng/mL or more, 250 ng/mL or more, 300 ng/mL or more, or 350 ng/mL or more. In a case in which the measurement system is a one factor system, although it is not limited, the content of the recombinant protein of factor C in the measurement sample at the time of endotoxin measurement can be set at 10 ng/mL or more, 20 ng/mL or more, 30 ng/mL or more, 40 ng/mL or more, 50 ng/mL or more, 60 ng/mL or more, 70 ng/mL or more, 100 ng/mL or more, 500 ng/mL or more, or 800 ng/mL or more.

The upper limit of the content of the recombinant protein of factor C in the measurement sample at the time of endotoxin measurement is not particularly limited, and is, for example, 10 mg/mL or less and is preferably less than 10 µg/mL (for example, 5 µg/mL or less or 2 µg/mL or less) from the viewpoint of production efficiency.

Furthermore, in the present specification, the content of the recombinant protein of factor C is a value calculated by ELISA method described in Examples.

The method for measuring endotoxin using a Limulus reagent is a method utilizing a progress of cascade reaction in which a serine protease precursor (for example, factor C, factor B, and a pro-clotting enzyme) contained in amoebocyte lysate is gradually activated in contact with endotoxin.

The endotoxin measuring method to which the enhancing method of the present invention is applied can be also a method in which the measurement is carried out by utilizing the protease activity of an active form factor C which is self-catalytically converted from a factor C to the active form (active form factor C) in contact with endotoxin (one factor system). Further, the endotoxin measuring method to be applied in the present invention may be a method of carrying out the measurement by utilizing the reaction of the conversion into an active form factor B as the factor B is cleaved off by the protease activity of the active form factor C which is a factor C self-catalytically converted to the active form in contact with endotoxin (two factor system). Furthermore, the endotoxin measuring method to be applied in the present invention may be a method of carrying out the measurement by utilizing, in addition to those series of reaction, a series of reaction which includes conversion into a clotting enzyme as a pro-clotting enzyme is cleaved off by the protease activity of the active form factor B (three factor system). The "cascade reaction" in the present specification includes the reactions of the one factor system, the two factor system, and the three factor system described above.

The endotoxin measuring method to which the enhancing method of the present invention is applied can be applied without being particularly limited, as long as it is an endotoxin measuring method which utilizes a recombinant reagent as an endotoxin measuring reagent using a recombinant protein of factor C. The recombinant reagent may be either a commercially available recombinant reagent or a reagent composed of a Limulus factor which has been produced by a well-known method.

With regard to the recombinant reagent, the factor C is a recombinant protein of Limulus factor prepared by genetic engineering techniques.

With regard to the above recombinant reagent, the factor B and a pro-clotting enzyme may be, each independently, either a natural Limulus factor obtained from a horseshoe crab, a recombinant protein of a Limulus factor which has been prepared by genetic engineering techniques, or a mixture containing, at an arbitrary ratio, a natural Limulus factor and a recombinant protein of Limulus factor. Preferably, the factor B and the pro-clotting enzyme are recombinant proteins of Limulus factor.

The natural Limulus factor may be those resulting from suitable purification and fractionation of a lysate which is obtained by a common method from hemocytes of a horseshoe crab as a raw material. Fractionation of Limulus factor can be carried out in view of the method described in the literature (Nakamura, T., Horiuchi, T., Morita, T., Iwanaga, S. (1986) J. Biochem. 99, 847-57), for example.

The recombinant protein of Limulus factor can be obtained by producing the Limulus factor in a cell to which a nucleic acid encoding the polypeptide of Limulus factor is introduced. The base sequence of the nucleic acid encoding Limulus factor can be obtained from a known database like NCBI (www.ncbi.nlm.nih.gov). Furthermore, the base sequence of the nucleic acid encoding Limulus factor can be also a variant of DNA of which codon combination is optimized for the expression in host cells.

The type of the horseshoe crab to be an origin of each Limulus factor is not particularly limited. As for the horseshoe crab, those belonging to genus Tachypleus, genus Limulus, or genus Carcinoscorpius can be exemplified. More specifically, four types, that is, Tachypleus tridentatus, Limulus polyphemus, Carcinoscorpius rotundicauda, and Tachypleus gigas, are known. These horseshoe crabs can be exemplified as a horseshoe crab to be an origin of Limulus factor. In addition, among those horseshoe crabs, Tachypleus tridentatus, Carcinoscorpius rotundicauda, or Limulus polyphemus is preferred, and Tachypleus tridentatus.is more preferred.

As for the polypeptide of Limulus factor, the polypeptides of (1) to (8) described in <Method for measuring endotoxin> described below are specifically exemplified. As for the nucleic acid encoding Limulus factor, base sequences encoding polypeptide of [1] to [7] described in <Method for measuring endotoxin> described below are specifically exemplified.

Production of Limulus factor using cells can be carried out by a well-known technique, and specific examples thereof include a method using mammalian cells or non-mammalian cells described in <Example 2> or the like described below (for example, insect cells, yeasts, parasitic protozoan such as Leishmania, or the like). Furthermore, production of Limulus factor using cells can be also carried out in view of the method described in the literature, for example (WO 2018/074498 A).

With regard to the enhancing method of the present invention, the method can be carried out under well-known conditions for measuring endotoxin except that the content of the recombinant protein of factor C at the time of endotoxin measurement is increased, for example, to an amount that is sufficient for the enhancement of sensitivity. The conditions for measurement are not limited, but, as for the pH of a reaction solution, the conditions with a pH of 5 to 10 and preferably 7 to 8.5 can be employed, for example. As for the reaction temperature, the conditions with a reaction temperature of 10°C to 80°C, preferably 20°C to 50°C, and more preferably 30°C to 40°C can be employed. As for the reaction temperature, 37°C is exemplified, for example. The reaction time is also not particularly limited, and may be suitably set depending on various conditions. The reaction time may be, for example, 5 minutes to 2 hours, preferably 15 minutes to 90 minutes, and more preferably 30 minutes to 40 minutes.

### <Method for measuring endotoxin>

Another aspect of the present invention is an endotoxin measuring method in which sensitivity of an endotoxin measuring reagent to the endotoxin of Helicobacter pylori is enhanced, in which the method includes preparing an endotoxin measurement sample containing the endotoxin measuring reagent and an analyte, the endotoxin measuring reagent contains a recombinant protein of horseshoe crab factor C, and enhancement of the sensitivity is achieved by increasing the concentration of the recombinant protein of factor C in the endotoxin measurement sample (hereinbelow, the endotoxin measuring method may be referred to as a "measurement method of the present invention"). Another embodiment of the present invention is a method for measuring endotoxin in which the method includes preparing an endotoxin measurement sample containing an endotoxin measuring reagent and an analyte, the endotoxin measuring reagent contains a recombinant protein of horseshoe crab factor C, and the concentration of the recombinant protein of factor C in the endotoxin measurement sample is increased to an amount that is sufficient for enhancing the sensitivity of the endotoxin measuring reagent to the endotoxin of Helicobacter pylori.

In the present invention, the measurement is used as a general description including detection, sensing, and quantification. Thus, the measurement method of the present invention may be a method for detecting endotoxin, a method for sensing endotoxin, or a method for quantifying endotoxin, for example.

The measurement method of the present invention is an endotoxin measuring method in which sensitivity of an endotoxin measuring reagent to the endotoxin of Helicobacter pylori is enhanced, in which the sensitivity is enhanced by increasing the concentration of a recombinant protein of factor C in an endotoxin measurement sample. With regard to the measurement method of the present invention, the method can be carried out under well-known conditions for measuring endotoxin except that the concentration of the recombinant protein of factor C is increased in an endotoxin measurement sample. The aspect of the concentration amount of the recombinant protein of factor C in an endotoxin measurement sample, which is sufficient for enhancing the sensitivity of an endotoxin measuring reagent to the endotoxin of Helicobacter pylori, is the same as the aspect of the content of the recombinant protein of factor C described in <Method for enhancing sensitivity of endotoxin measuring reagent> above.

Hereinbelow, details of the measurement method are described.

The measurement method of the present invention is an endotoxin measuring method including the following steps (A) and (B) :
(A) a step of mixing a recombinant protein of horseshoe crab factor C and an analyte; and
(b) a step of measuring protease activity of Limulus factor.

The above step (A) is a step of mixing a recombinant protein of horseshoe crab factor C and an analyte. In a case in which the analyte is a sample containing endotoxin, a factor C contacted with the endotoxin converts into an active form factor C.

With regard to the above step (A), as long as an operation of mixing a recombinant protein of factor C and an analyte is included, it may further include an operation of mixing other materials. Examples of "other materials" described herein include a factor B, a pro-clotting enzyme, and a compound for detection (substrate for detection). In a case in which the above step (A) is a step of mixing a recombinant protein of factor C, a factor B, a pro-clotting enzyme, and an analyte and also the analyte is a sample containing endotoxin, a cascade reaction by which a factor C brought into contact with the endotoxin converts into an active form factor C, a factor B converts into an active form factor B, and a pro-clotting enzyme converts into a clotting enzyme progresses.

In the present invention, the "factor C" is not particularly limited as long as it is a factor C having the function of a horseshoe crab factor C. Definition of the "horseshoe crab" described herein is the same as the description given in <Method for enhancing sensitivity of endotoxin measuring reagent> described above. The expression of "function of a horseshoe crab factor C" means the function of a horseshoe crab factor C as a protease precursor. The expression "function of a horseshoe crab factor C" specifically means the function of conversion into an active form (active form factor C) in the co-existence of endotoxin and expression of the protease activity. The function of a horseshoe crab factor C is a function of conversion into an active form (active form factor C) in the co-existence of endotoxin and conversion into an active form (active form factor B) by cleaving off the factor B, for example. Furthermore, the function of a horseshoe crab factor C is a function of conversion into an active form (active form factor C) in the co-existence of endotoxin and release of a labeling material by cleaving off a compound for detection, which becomes a substrate of the active form factor C, for example. As for the compound for detection described herein, the aspect described in the followings can be suitably used, for example.

In the present invention, the "factor B" is not particularly limited as long as it is a factor B having the function of a horseshoe crab factor B. Definition of the "horseshoe crab" described herein is the same as the description given in <Method for enhancing sensitivity of endotoxin measuring reagent> described above. The expression of "function of a horseshoe crab factor B" specifically means the function of conversion into an active form (active form factor B) in accordance with a contact with an active form factor C and expression of the protease activity. The function of a horseshoe crab factor B is a function of conversion into an active form (active form factor B) in accordance with a contact with an active form factor C and conversion into a clotting enzyme by cleaving off a pro-clotting enzyme, for example. Furthermore, the function of a horseshoe crab factor B is a function of conversion into an active form (active form factor B) in accordance with a contact with an active form factor C and release of a labeling material by cleaving off a compound for detection, which becomes a substrate of the active form factor B, for example. As for the compound for detection described herein, the aspect described in the followings can be suitably used, for example.

The "pro-clotting enzyme" described in the present invention is not particularly limited as long as it is a pro-clotting enzyme having the function of a horseshoe crab pro-clotting enzyme. Definition of the "horseshoe crab" described herein is the same as the description given in <Method for enhancing sensitivity of endotoxin measuring reagent> described above. The expression of "function of a horseshoe crab pro-clotting enzyme" means the function of a horseshoe crab pro-clotting enzyme as a protease precursor. The expression of "function of a horseshoe crab pro-clotting enzyme" specifically means the function of conversion into an active form (clotting enzyme) in the co-existence of an active form factor B and expression of the protease activity. The function of a horseshoe crab pro-clotting enzyme indicates a function of conversion into an active form (clotting enzyme) in the co-existence of an active form factor B and forming of coagulin gel by cleaving off coagulogen, for example. Furthermore, the function of a horseshoe crab pro-clotting enzyme is a function of conversion into an active form (clotting enzyme) in the co-existence of an active form factor B and release of a labeling material by cleaving off a compound for detection, which becomes a substrate of the clotting enzyme, for example. As for the compound for detection described herein, the aspect described in the followings can be suitably used, for example.

As for the factor C of the present invention, specifically, the polypeptide expressed in any one of the following (1) to (4) is exemplified.
(1) Polypeptide having the amino acid sequence represented by SEQ ID NO: 2.
(2) Polypeptide having the amino acid sequence represented by SEQ ID NO: 4.
(3) Polypeptide having the amino acid sequence represented by SEQ ID NO: 12.
(4) Polypeptide having an amino acid sequence in which one or plural amino acid residues of the amino acid sequence shown in any one of the above (1) to (3) are substituted, deleted, inserted, and/or added, and also having the function of a horseshoe crab factor C.
   As for the factor B of the present invention, specifically, the polypeptide expressed in any one of the following (5) and (6) is exemplified.
(5) Polypeptide having the amino acid sequence represented by SEQ ID NO: 6.
(6) Polypeptide having an amino acid sequence in which one or plural amino acid residues of the amino acid sequence shown in the above (5) are substituted, deleted, inserted, and/or added, and also having the function of a horseshoe crab factor B.
   As for the pro-clotting enzyme of the present invention, specifically, the polypeptide expressed in any one of the following (7) and (8) is exemplified.
(7) Polypeptide having the amino acid sequence represented by SEQ ID NO: 8.
(8) Polypeptide having an amino acid sequence in which one or plural amino acid residues of the amino acid sequence shown in the above (7) are substituted, deleted, inserted, and/or added, and also having the function of a horseshoe crab pro-clotting enzyme.

The expression "plural" described in the above (4), (6), and (8) means the number (total number) of amino acid residues that are substituted, deleted, inserted, and/or added to the extent that allows no functional loss of the polypeptides as Limulus factor. The expression "plural" may be the number of preferably 10% or less, more preferably 5% or less, even more preferably 2% or less, particularly preferably 1% or less, and most preferably 0.5% or less relative to the total number of amino acid residues constituting the polypeptide, for example.

Accordingly, since the total number of amino acid residues is 1,019 in the case of the amino acid sequence of the polypeptide shown in the above (4), the "plural" may be preferably 2 to 100, more preferably 2 to 50, even more preferably 2 to 20, particularly preferably 2 to 10, and most preferably 2 to 5. The expression "plural" described in the above (4), (6), and (8) may be, as a specific individual number, an integer of 2, 3, 4, 5, or the like.

The expression "substituted, deleted, inserted, and/or added" described in the above (4), (6), and (8) indicates a conservative mutation, for example. Representative example of the conservative mutation is a conservative substitution. The conservative substitution means a mutation for having a substitution among Phe, Trp, and Tyr when an aromatic amino acid is present at the substitution site, a substitution among Leu, Ile, and Val when a hydrophobic amino acid is present at the substitution site, a substitution between Gln and Asn when a polar amino acid is present at the substitution site, a substitution among Lys, Arg, and His when a basic amino acid is present at the substitution site, a substitution between Asp and Glu when an acidic amino acid is present at the substitution site, or a substitution between Ser and Thr when an amino acid having hydroxyl group is present at the substitution site. Specific examples of the substitution which is regarded as conservative mutation includes a substitution of Ser or Thr for Ala, a substitution of Gln, His, or Lys for Arg, a substitution of Glu, Gln, Lys, His, or Asp for Asn, a substitution of Asn, Glu, or Gln for Asp, a substitution of Ser or Ala for Cys, a substitution of Asn, Glu, Lys, His, Asp, or Arg for Gln, a substitution of Gly, Asn, Gln, Lys, or Asp for Glu, a substitution of Pro for Gly, a substitution of Asn, Lys, Gln, Arg, or Tyr for His, a substitution of Leu, Met, Val, or Phe for Ile, a substitution of Ile, Met, Val, or Phe for Leu, a substitution of Asn, Glu, Gln, His, or Arg for Lys, a substitution of Ile, Leu, Val, or Phe for Met, a substitution of Trp, Tyr, Met, Ile, or Leu for Phe, a substitution of Thr or Ala for Ser, a substitution of Ser or Ala for Thr, a substitution of Phe or Tyr for Trp, a substitution of His, Phe, or Trp for Tyr, and a substitution of Met, Ile, or Leu for Val.

Each of the polypeptides described in the above (4), (6), and (8) has similarity of preferably 85% or higher, more preferably 90% or higher, even more preferably 95% or higher, and particularly preferably 99% or higher to the amino acid sequences of the polypeptides described in the above (1), (2), (3), (5), and (7), for example, and each of the polypeptides may be also a polypeptide having the function of Limulus factor. Furthermore, the "similarity" described herein is a concept which includes "identity", and thus an application can be made to a suitable aspect of the polypeptide by interchanging the similarity with identity.

The above polypeptides can be prepared by a well-known method on the basis of the descriptions of the present specification. For example, the above polypeptides can be prepared by genetic engineering techniques. Specifically, the polypeptides can be obtained by artificially introducing a DNA encoding the above polypeptide to host cells and expressing the polypeptide therein.

Examples of the DNA encoding the above polypeptides include the followings.

[1] DNA having the base sequence represented by SEQ ID NO: 1.
[2] DNA having the base sequence represented by SEQ ID NO: 3.
[3] DNA having the base sequence represented by SEQ ID NO: 5.
[4] DNA having the base sequence represented by SEQ ID NO: 7.
[5] DNA having the base sequence represented by SEQ ID NO: 9.
[6] DNA having the base sequence represented by SEQ ID NO: 10.
[7] DNA having the base sequence represented by SEQ ID NO: 11.

The above polypeptides may be prepared as a culture solution itself obtained by culturing cells, or may be prepared as a fraction obtained by purifying the culture solution to desired extent, for example.

The "compound for detection" described in the present invention is a substrate which is used for measuring the presence or absence or the amount of activated Limulus factor or progress of the cascade reaction. The compound for detection may be a substrate for measuring an active form factor C, a substrate for measuring an active form factor B, or a substrate for measuring a clotting enzyme. The compound for detection is not particularly limited as long as it is a substrate of activated Limulus factor. The compound for detection may be either a protein, a peptide, or a derivative thereof, for example.

The protein may be either a natural protein or a recombinant protein. As for the protein, coagulogen as a substrate of the clotting enzyme is exemplified. The natural coagulogen can be prepared by fractionation from a lysate, for example. Furthermore, the recombinant coagulogen can be prepared in view of the method described in the literature (Miyata et al., Separate Issue of Proteins, Nucleic acids, and Enzymes, No. 29; P30-43; 1986), for example.

The peptide may be a synthetic substrate obtained by chemical synthesis, for example. The synthetic substrate is not particularly limited as long as it is a substrate suitable for measuring the presence or absence or the amount of activated Limulus factor or the progress of the cascade reaction. The synthetic substrate is preferably a peptide derivative.

As for the synthetic substrate, a substrate represented by the general formula Y-X-Z (in the formula, Y may or may not be present, and, when Y is present, Y is a protecting group (namely, Y is a hydrogen atom or a protecting group), X is a peptide, and Z is a labeling material) is exemplified. It is preferable that this synthetic substrate has a property of cleaving off the covalent bond between X and Z by activated Limulus factor and releasing the labeling material Z. In the above general formula, Y is preferably a protecting group for the amino group at the N terminal of a peptide. In the above general formula, the bond between Y and X is preferably an amide bond which is formed between the carboxy group of a protecting group and an α amino group at the N terminal of a peptide. Furthermore, in the above general formula, the bond between X and Z is preferably an amide bond which is formed between the carboxy group at the C terminal of a peptide and an amino group of the labeling material Z.

The protecting group as Y is not particularly limited, and a well-known protecting group which is applicable for peptide protection can be used. Examples of the protecting group include a tert-butoxycarbonyl group (Boc), a benzyloxycarbonyl group (Cbz), a benzyl group (Bzl), a benzoyl group (Bz), and an acetyl group (Ac).

The peptide (X) is not particularly limited as long as it is a peptide having an amino acid sequence that is a substrate of activated Limulus factor. The peptide is preferably a substrate that is suitable for measuring serine protease, and is preferably a peptide having an Arg (R) residue at the C terminal.

In a case in which the activated Limulus factor is a factor C, the peptide is preferably a peptide having an amino acid sequence represented by the general formula X-Pro-Arg (in the formula, X represents an arbitrary amino acid). Specifically, in a case in which the activated Limulus factor is a factor C, the peptide is preferably a peptide having an amino acid sequence represented by Val-Pro-Arg (VPR) or Asp-Pro-Arg (DPR).

In a case in which the activated Limulus factor is a factor B, the peptide is preferably a peptide having an amino acid sequence represented by the general formula X-Thr-Arg (in the formula, X represents an arbitrary amino acid). Specifically, in a case in which the activated Limulus factor is a factor B, the peptide is preferably a peptide having an amino acid sequence represented by Leu-Thr-Arg (LTR) or Met-Thr-Arg (MTR).

In a case in which the activated Limulus factor is a pro-clotting enzyme, the peptide is preferably a peptide having an amino acid sequence represented by the general formula X-Gly-Arg (in the formula, X represents an arbitrary amino acid). Specifically, in a case in which the activated Limulus factor is a pro-clotting enzyme, the peptide is preferably a peptide having an amino acid sequence represented by Leu-Gly-Arg (LGR) or Glu-Gly-Arg (EGR) .

The labeling material (Z) is not particularly limited, and a well-known labeling material applicable for measurement of protease activity can be used. As a labeling material, a compound allowing optical detection (of color, fluorescence, light emission, or the like) upon release from a peptide can be used, for example. Examples of such labeling material include para-nitroaniline (pNA), 7-methoxycoumarine-4-acetic acid, 2,4-dinitroaniline (DNP), 7-amino-4-methylcoumarin (AMC), and 7-amino-4-trifluoromethyl coumarin. Furthermore, as a labeling material, a compound allowing detection by an electrochemical measurement method (voltammetry, amperometry, or the like) upon release from a peptide can be used, for example. Examples of such a labeling material include p-aminophenol (pAP), p-methoxyaniline (pMA), N-methyl-p-phenylenediamine (MPDD), and N,N'-dimethyl-p-phenylenediamine (DMPD).

In the step (A) of the above measurement method of the present invention, the Limulus factor, the analyte, the compound for detection, and other materials (buffering reagent or the like) may be added at any order and mixed with one another. For example, in the above step (A), the analyte may be added to a mixture of the Limulus factor, the compound for detection, and other materials and mixed therein. Furthermore, for example, in the above step (A), a mixture of the Limulus factor, the compound for detection, and other materials may be added to the analyte and mixed therein. Mixing in the step (A) may be carried out inside of a vessel (in a vessel) having an opening on one end (test tube, vial, or the like), for example. The analyte is not particularly limited as long as it is a sample from which endotoxin detection is required, and examples thereof include, in addition to water for injection, pharmaceutical product, transfusion solution, blood preparation, medical device (medical instrument), quasi drug, cosmetic product or the like, food product, environmental sample such as water, air, river, or soil, natural protein, gene recombinant protein, nucleic acid, enzyme, carbohydrate, and electrolyte, components of a living body like blood, body fluid, and tissues.

The above step (B) is a step of measuring protease activity of the polypeptide to be used in the present invention. This step is a step of measuring a labeling material released from a compound for detection, for example. During this step, the labeling material is released, in an amount (mole number) according to the protease activity (total activity) of an active form Limulus factor, from a compound for detection, and therefore protease activity of the polypeptide to be used in the present invention can be measured by measuring the labeling material released from a compound for detection. The labeling material released from a compound for detection can be measured, for example, by a photometric device such as a spectrophotometer or a fluorometer. Furthermore, the labeling material released from a compound for detection can be measured, for example, by an electrochemical measurement device such as a voltammetric device or an amperometric device. For example, by comparing the measurement value shown from the above step with a blank value (measurement value obtained by having an endotoxin-free analyte as a measurement subject), the presence or absence of endotoxin in an analyte can be determined. Furthermore, the measurement method of the present invention may be a step for determining the presence or absence of endotoxin in an analyte by determining the presence or absence of gelation of a mixture solution, for example.

The measurement method of the present invention may include another step in addition to the above steps (A) and (B). The measurement method of the present invention may include a step for determining the presence or absence of endotoxin in an analyte by comparing the measurement value obtained from the step (B) with a blank value. Furthermore, the measurement method of the present invention may include a step for converting the measurement value obtained from the step (B) into other value, for example. As a step for converting the measurement value into other value, a step of calculating the amount of endotoxin on the basis of measurement value is exemplified, for example. Specifically, this step is a step of converting the measurement value that is obtained from measurement of an analyte into the amount of endotoxin on the basis of relationship (standard curve) between the measurement value obtained from measurement by replacing an analyte with a standard material with known concentration and the concentration of standard material, for example.

The Limulus reaction in the measurement method of the present invention is preferably carried out in an aqueous solvent.

### <Endotoxin measuring reagent>

In another aspect of the present invention, there is provided an endotoxin measuring reagent containing a horseshoe crab factor C. The horseshoe crab factor C contained in the endotoxin measuring reagent according to the present invention is the following (a) and may further contain (b) and/or (c), and the present invention may also include an aspect of the endotoxin measuring reagent in which relative potency of the endotoxin of Helicobacter pylori is 0.1 times or more relative potency measured by using a lysate reagent:
(a) a recombinant protein of horseshoe crab factor C;
(b) a recombinant protein of horseshoe crab factor B; and
(c) a recombinant protein of horseshoe crab pro-clotting enzyme.

Herein, the aspect of the recombinant protein of the Limulus factor of (a), (b), and (c) is the same as the aspect of <Method for measuring endotoxin> described above.

The lysate reagent may be, for example, the lysate reagent mentioned above in <Method for enhancing sensitivity of endotoxin measuring reagent> described above.

The endotoxin measuring reagent can be suitably used for carrying out the measurement method of the present invention.

As it has been described in the above, when the relative potency of the endotoxin of Helicobacter pylori measured by using an endotoxin measuring reagent does not correspond to desired potency, the relative potency of the endotoxin of Helicobacter pylori can be enhanced by increasing the content of the recombinant protein of factor C at the time of endotoxin measurement.

As another aspect, the present invention includes at least the above (a) and may further include (b) and/or (c), and the endotoxin measuring reagent can be an endotoxin measuring reagent in which relative potency (measured by a colorimetric method, a fluorescence method, or the like) of the endotoxin of Helicobacter pylori is 200 (EU/µg) or higher, preferably 250 (EU/µg) or higher, and more preferably 300 (EU/µg) or higher.

Herein, the aspect of the recombinant protein of the Limulus factor of (a), (b), and (c) is the same as the aspect of <Method for measuring endotoxin> described above.

The relative potency of the endotoxin of Helicobacter pylori may be, for example, 200 (EU/µg) or higher and 3,000 (EU/µg) or lower, 250 (EU/µg) or higher and 2,500 (EU/µg) or lower, or 300 (EU/µg) or higher and 2,000 (EU/µg) or lower. In an embodiment, the endotoxin measuring reagent contains a recombinant protein of factor C in an amount at which the relative activity is achieved. The relative potency of the endotoxin of Helicobacter pylori is a value measured using a measurement sample containing the endotoxin of Helicobacter pylori at a concentration equal to or more than the lower limit of quantitation.

Furthermore, the present aspect may be a specific example of the aspect in which the relative potency of the endotoxin of Helicobacter pylori is 0.1 times or more the relative potency measured with the lysate reagent.

The endotoxin measuring reagent may contain other configurations as long as the above components are contained as a constitutional component. Examples of other configurations described herein include a compound for detection, an instruction for use describing the product information or the like. The instruction for use may clearly describe that, when the relative potency of the endotoxin of Helicobacter pylori of an endotoxin measuring reagent does not correspond to the desired potency, the content of the recombinant protein of factor C at the time of endotoxin measurement is increased to an amount that is sufficient for enhancement of the sensitivity.

In an embodiment, the endotoxin measuring reagent is based on a three factor system and contains a compound for detection represented by the above general formula Y-X-Z (in the formula, Y is a hydrogen atom or a protecting group, X is a peptide containing the amino acid sequence, which is a substrate of a pro-clotting enzyme, and Z becomes a labeling material which is optically detectable when being released from X). Examples of Z may include para-nitroaniline, 7-methoxycoumarine-4-acetic acid, 7-amino-4-methylcoumarin, and 7-amino-4-trifluoromethyl coumarin. In a specific embodiment, Z is a labeling material, which is detectable as coloring when being released from X, and is preferably para-nitroaniline.

In an embodiment, the endotoxin measuring reagent is based on a two factor system and contains a compound for detection represented by the above general formula Y-X-Z (in the formula, Y is a hydrogen atom or a protecting group, X is a peptide containing the amino acid sequence, which is a substrate of a factor B, and Z becomes a labeling material which is optically detectable when being released from X). Examples of Z may include para-nitroaniline, 7-methoxycoumarine-4-acetic acid, 7-amino-4-methylcoumarin, and 7-amino-4-trifluoromethyl coumarin. In a specific embodiment, Z is a labeling material, which is detectable as fluorescence when being released from X, and is preferably 7-methoxycoumarine-4-acetic acid, 7-amino-4-methylcoumarin, or 7-amino-4-trifluoromethyl coumarin.

In an embodiment, the endotoxin measuring reagent is based on a one factor system and contains a compound for detection represented by the above general formula Y-X-Z (in the formula, Y is a hydrogen atom or a protecting group, X is a peptide containing the amino acid sequence, which is a substrate of a factor C, and Z becomes a labeling material which is optically detectable when being released from X). Examples of Z may include para-nitroaniline, 7-methoxycoumarine-4-acetic acid, 7-amino-4-methylcoumarin, and 7-amino-4-trifluoromethyl coumarin. In a specific embodiment, Z is a labeling material, which is detectable as fluorescence when being released from X, and is preferably 7-methoxycoumarine-4-acetic acid, 7-amino-4-methylcoumarin, or 7-amino-4-trifluoromethyl coumarin. Furthermore, in the case of a one factor system, as compared to a two factor system or a three factor system, the relative potency of 200 EU/µg or higher of the endotoxin of Helicobacter pylori can be achieved even with a small amount of the recombinant protein of factor C.

The Limulus factor in the above endotoxin measuring reagent is preferably provided as a freeze-dried product.

One aspect of the present invention relates to an endotoxin measuring reagent having enhanced sensitivity to the endotoxin of Helicobacter pylori.

Another aspect of the present invention is a method for producing an endotoxin measuring reagent having enhanced sensitivity to the endotoxin of Helicobacter pylori, in which the endotoxin measuring reagent contains a recombinant protein of horseshoe crab factor C and includes increasing the content of the recombinant protein of factor C in the endotoxin measuring reagent (hereinbelow, the method may be referred to as a "production method of the present invention"). According to an embodiment of the present invention, the content of the recombinant protein of factor C in the endotoxin measuring reagent is increased to an amount that is sufficient for enhancing the sensitivity.

With regard to the aspect relating to the content of the recombinant protein of factor C in the endotoxin measuring reagent that is sufficient for enhancement of the sensitivity, for example, the content can be set so as to be an aspect of the content of the recombinant protein factor C in a measurement sample at the time of endotoxin measurement described in <Method for enhancing sensitivity of endotoxin measuring reagent> above. Specifically, the content can be varied depending on desired relative potency, measurement system, hosts of the recombinant protein of factor C, or the like. In a case in which the measurement system is based on three factor system, although it is not limited, the content of the recombinant protein of factor C in an endotoxin measuring reagent (in an amount of single use) can be set, in terms of the concentration in a measurement sample at the time of endotoxin measurement, at 140 ng/mL or more, 162 ng/mL or more, 180 ng/mL or more, 200 ng/mL or more, 250 ng/mL or more, 300 ng/mL or more, 400 ng/mL or more, 500 ng/mL or more, 600 ng/mL or more, or 700 ng/mL or more. In a case in which the measurement system is a two factor system, although it is not limited, the content of the recombinant protein of factor C in an endotoxin measuring reagent (in an amount of single use) can be set, in terms of the concentration in a measurement sample at the time of endotoxin measurement, at 235 ng/mL or more, 250 ng/mL or more, 300 ng/mL or more, or 350 ng/mL or more as the concentration at the time of endotoxin measurement. In a case in which the measurement system is a one factor system, although it is not limited, the content of the recombinant protein of factor C in an endotoxin measuring reagent (in an amount of single use) can be set, in terms of the concentration in a measurement sample at the time of endotoxin measurement, at 10 ng/mL or more, 20 ng/mL or more, 30 ng/mL or more, 40 ng/mL or more, 50 ng/mL or more, 60 ng/mL or more, 70 ng/mL or more, 100 ng/mL or more, 500 ng/mL or more, or 800 ng/mL or more.

The upper limit of the content of the recombinant protein of factor C in the measurement sample at the time of endotoxin measurement is not particularly limited, and is, for example, 10 mg/mL or less and is preferably less than 10 µg/mL (for example, 5 µg/mL or less or 2 µg/mL or less) from the viewpoint of production efficiency.

The measuring reagent of the present invention and the endotoxin measuring reagent produced by the production method of the present invention can be suitably used for carrying out the measurement method of the present invention.

As the content of the recombinant protein of factor C is increased in the measuring reagent of the present invention and the endotoxin measuring reagent produced by the production method of the present invention, the relative potency of the endotoxin of Helicobacter pylori measured by using the endotoxin measuring reagent is enhanced.

The measuring reagent of the present invention and the endotoxin measuring reagent produced by the production method of the present invention may further contain other configurations as long as a recombinant protein of factor C is contained therein as a constitutional component. Examples of other configurations described herein include a factor B, a pro-clotting enzyme, a compound for detection, and an instruction for use describing the product information. The aspect of the recombinant protein of factor C and the above other constitutional components are the same as the aspect of the recombinant protein of factor C and other configurations that are described in <Method for enhancing sensitivity of endotoxin measuring reagent> and <Method for measuring endotoxin> above. In an embodiment, the endotoxin measuring reagent does not substantially contain a surfactant.

According to the production method of the present invention, the production can be made by a well-known method for producing a recombinant reagent except that the content of the recombinant protein of factor C is increased in the endotoxin measuring reagent. Namely, by combining a recombinant protein of factor C with the above other constitutional components, the endotoxin measuring reagent can be produced.

### <Kit for endotoxin measurement>

Furthermore, the present invention may also include an aspect of a kit for endotoxin measurement that is characterized by including the above endotoxin measuring reagent as a constitutional product.

The kit can be suitably used for carrying out the measurement method of the present invention.

Furthermore, the kit may further include other constitutional products as long as it includes the above endotoxin measuring reagent as a constitutional product. Examples of other constitutional products described herein include a compound for detection, a buffering reagent, distilled water, Reference Standard Endotoxin, a micro plate, and an instruction for use describing the product information.

The kit may contain each constitutional product that is separately present, or may contain a mixture obtained by combining arbitrarily each constitutional product. For example, the kit may contain each Limulus factor that is separately present, may contain Limulus factors according to an aspect in which Limulus factors have been mixed in advance, or may further contain a compound for detection according to an aspect in which the compound has been mixed in advance.

### Examples

Hereinbelow, the present invention is specifically described in view of Examples, but these are merely exemplifications of the present invention and the scope of the present invention is not limited thereto.

### <Example 1: Comparison of sensitivity to endotoxin among reagents>

By employing a recombinant reagent and a lysate reagent, comparison was made to see any difference in the sensitivity to endotoxins of different origin.

The following endotoxin measuring reagents were prepared. Furthermore, the "recombinant reagent" indicates a preparation which contains a factor C produced by genetic recombination techniques, and the "lysate reagent" indicates a preparation which contains a natural factor C prepared from hemocyte extract components of a horseshoe crab.

(1) Preparation containing recombinant factor C (rFC), recombinant factor B (rFB), recombinant pro-clotting enzyme (rPCE), compound for detection (synthetic substrate Boc-LGR-pNA; PEPTIDE INSTITUTE, INC.) and additives of PyroSmart (registered trademark): commercially available recombinant reagent A
(2) PyroGene (LONZA JAPAN): commercially available recombinant reagent B
(3) EndoZyme (registered trademark) II (bioMérieux Japan Ltd.): commercially available recombinant reagent C
(4) Endospecy (registered trademark) ES-50M (SEIKAGAKU CORPORATION): commercially available lysate reagent A
(5) Kinetic QCL (LONZA JAPAN): commercially available lysate reagent B

As an endotoxin to be measured, endotoxins of two types, Helicobacter pylori CA2 (LPS Research Institute) and Salmonella minnesota R595Re (List Biological Laboratories, Inc.), were used. Furthermore, as Reference Standard Endotoxin(RSE) derived from Escherichia coli 0113: H10, Reference Standard Endotoxin of Japanese Pharmacopoeia (Pharmaceutical and Medical Device Regulatory Science Society of Japan) or US Pharmacopoeia (SEIKAGAKU CORPORATION) was used.

Endotoxin of Helicobacter pylori CA2 was dissolved in water for injection (Otsuka Pharmaceutical Factory, Inc.) to have 1 mg/mL. Endotoxin of Salmonella minnesota R595Re was dissolved to have 1 mg/mL using 0.1% (v/v) triethylamine (FUJIFILM Wako Pure Chemical Corporation), and then neutralized with Tris buffer solution (FUJIFILM Wako Pure Chemical Corporation). The stock solution of those endotoxins was diluted with water for injection such that the endotoxin concentration falls within the quantification range described in the instruction for use for each reagent, and thus a diluted solution of endotoxin was obtained.

For the measurement using the commercially available recombinant reagent A, the preparation was mixed, in a micro plate, with water for injection (blank) or a diluted solution of endotoxin to prepare a measurement sample. According to this assay system, a factor C (FC) is activated by endotoxin to produce an active form FC, a factor B (FB) is activated by the active form FC to produce an active form FB, a pro-clotting enzyme (PCE) is activated by the active form FB to produce a clotting enzyme, and synthetic substrate Boc-LGR-pNA is cleaved off by the clotting enzyme to release para-nitroaniline. By measuring the absorbance of a measurement sample using an absorbance microplate reader (for 30 minutes at 37°C, with a main wavelength of 405 nm and a reference wavelength of 492 nm and an interval of 15 seconds), the absorbance change ratio per unit time (for 1 minute) was calculated (mAbs/min). Subsequently, from a calibration curve established with RSE, endotoxin activity (EU/mL) of a diluted solution of endotoxin at each concentration was calculated, and, by dividing the calculated value by each concentration of diluted solution of endotoxin, relative potency (EU/µg) at each endotoxin concentration was calculated. Among the obtained relative potencies, values falling within the quantification range of RSE were averaged to determine the relative potency of endotoxin (Table 1).

For other recombinant reagents (commercially available recombinant reagent B, commercially available recombinant reagent C) and lysate reagents (commercially available lysate reagent A, commercially available lysate reagent B), the relative potency was also determined in the same manner as that for the commercially available recombinant reagent A on the basis of the results measured according to an instruction for use (Table 1).

As a result, the relative potency of endotoxin of Helicobacter pylori CA2, which has been obtained from the commercially available recombinant reagents A, B, and C, was 147.3, 3.21, and 0.07 EU/µg, respectively. Those values were significantly lower than the relative potency obtained by using a lysate reagent (about 2,000 EU/µg), and the difference was 14 times or more (Table 1). Namely, a huge difference was seen in terms of the sensitivity to endotoxin of Helicobacter pylori CA2 between the commercially available recombinant reagent and lysate reagent. On the other hand, the relative potency of endotoxin of Salmonella minnesota R595Re showed no huge difference between the commercially available recombinant reagent and lysate reagent (Table 1).

Furthermore, the same test as above was carried out by using endotoxin of Escherichia coli 055: B5 and Salmonella typhimulium. However, the relative potency of those endotoxins also showed no huge difference between the commercially available recombinant reagent and lysate reagent.

**Table 1**

| | Relative potency (EU/µg) when RSE is taken as reference | |
|---|---|---|
| | H. pylori | S. minnesota |
| Commercially available recombinant reagent A | 147.3 | 54448 |
| Commercially available recombinant reagent B | 3.21 | 60027 |
| Commercially available recombinant reagent C | 0.07 | 28329 |
| Commercially available lysate reagent A | 2193 | 43240 |
| Commercially available lysate reagent B | 1971 | 54484 |

### <Example 2: Influence of rFC content on sensitivity to endotoxin of Helicobacter pylori CA2>

### A. Preparation of recombinant protein

According to Mizumura H, Ogura N, Aketagawa J, Aizawa M, Kobayashi Y, Kawabata SI, Oda T. Innate Immun. 2017 Feb; 23 (2): 136-146, or "Recombinant proteins derived from limulus bacterium, and DNA molecules encoding same" (WO 2018/074498 A1), rFC, rFB, and rPCE of Tachypleus tridentatus (T.t.) were expressed as described below to obtain various kinds of enzyme solution. Furthermore, SEQ ID NO: 1 was used as DNA encoding FC of T.t., SEQ ID NO: 5 or 9 was used as DNA encoding FB of T.t., and SEQ ID NO: 7 was used as DNA encoding PCE of T.t.

### (1) Stable expression of rFC using mammalian cells (CHO DG44 cells)

Chinese hamster ovarian cell line (CHO DG44 cells) was used as a host, and an enzyme solution of rFC was prepared according to the following procedure.

DNA (SEQ ID NO: 1) encoding FC of T.t. was inserted between the EcoRI and XbaI recognition sites of an expression vector (pCI-neo; manufactured by Promega Corporation) to produce a plasmid for FC expression. The plasmid for FC expression and a plasmid for expressing dehydrofolate reductase (dhfr) were introduced to CHO DG44 cells by using a lipofection reagent (Lipofectamine LTX; Life Technologies Corporation). The cells were subjected to static culture under supply of 5 (v/v)% CO₂ at 37°C, and the cells having the expression plasmid introduced onto the genome were selected with geneticin (Invitrogen) to obtain a cell line highly expressing rFC from the poly clones. From polyclonal cell group which has been acclimated in a medium containing 5 µM methotrexate (MTX), the cell line highly expressing rFC was monoclonized. The cell line (mono clone) was floated after undergoing acclimation in serum-free complete synthetic medium. From the floating culture solution in which cells were grown to later stage of logarithmic growth phase under supply of 5 (v/v)% CO₂ at 37°C, culture supernatant was obtained by centrifugation (3,000 ×g, for 30 minutes, 4°C) to prepare an enzyme solution of rFC.

### (2) Stable expression of rFC using insect cells (Sf9 cells)

An enzyme solution of rFC was prepared according to the following procedure, by using insect cells (Sf9 cells) as host cells.

DNA (SEQ ID NO: 1) encoding FC of T.t. was inserted between the EcoRV and MluI recognition sites of an expression vector (pIZ-V5; Life Technologies Corporation) to produce a plasmid for FC expression. The plasmid for FC expression was introduced to Sf9 cells by using a cellfectin reagent (Life Technologies Corporation). The cells were subjected to static culture at 28°C, and the cells having the expression plasmid introduced onto the genome were selected against zeocin (Invitrogen). After that, candidates in which the factor C gene is stably expressed were isolated by a cloning cylinder method, and Sf9 cell line highly expressing rFC was selected. The Sf9 cell line highly expressing rFC was then subjected to floating culture (28°C) to later stage of logarithmic growth phase (6 to 8 x 10⁶ cells/mL) in Sf900III medium containing 1x penicillin/streptomycin (Life Technologies Corporation) and 50 µg/mL zeocin. Culture supernatant was obtained by centrifugation (3,000 ×g, for 30 minutes, 4°C) of the culture solution to prepare an enzyme solution of rFC.

### (3) Stable expression of rFB and rPCE using insect cells (Sf9 cells)

Insect cells (Sf9 cells) were used as a host, and enzyme solutions of rFB and rPCE were prepared according to the following procedure.

DNA (SEQ ID NO: 7, 9) encoding FB and PCE of T.t. was inserted between the EcoRV and MluI recognition sites of an expression vector (pIZ-V5; Life Technologies Corporation) to produce each of a plasmid for FB expression and a plasmid for PCE expression. Furthermore, the base sequence represented by SEQ ID NO: 9 is a base sequence in which the base sequence represented by SEQ ID NO: 5 is optimized for expression in insect cells. According to the same method as FC above, enzyme solutions of rFB and rPCE were prepared. The protein concentration in the enzyme solutions of rFB and rPCE was measured by using a protein assay kit (Bio-Rad Laboratories, Inc.).

### (4) Transient expression of rFC using Expi CHO Expression System

By using Expi CHO Expression System (Thermo Fisher Scientific Inc.), an enzyme solution of rFC was prepared according to the attached instructions.

DNA (SEQ ID NO: 1) encoding FC of T.t. was inserted between the EcoRI and XbaI recognition sites of an expression vector (pCI-neo; manufactured by Promega Corporation) to produce a plasmid for FC expression. The plasmid for FC expression which has been mixed with ExpiFectamin CHO was introduced to Expi CHO-S cells in the co-existence of Expi CHO Expression medium. The cells were grown in serum-free complete synthetic medium under supply of 8 (v/v)% CO₂ at 37°C. The floating culture solution was subjected to centrifugation (3,000 ×g, for 30 minutes, 4°C) to obtain culture supernatant, and thus an enzyme solution of rFC was prepared.

### B. Measurement of rFC concentration

The rFC concentration in a sample was calculated by ELISA method using anti FC antibody in view of Kobayashi Y, Shiga T, Shibata T, Sako M, Maenaka K, Koshiba T, Mizumura H, Oda T, Kawabata S. J Biol Chem. 2014 Sep 12; 289 (37): 25987-95. Specifically, 2C12 monoclonal antibody (mouse), which is an anti FC antibody, was used as a capture antibody, anti FC polyclonal antibody (rabbit) was used as a detection antibody, and horseradish peroxidase (HRP)-labeled anti rabbit IgG polyclonal antibody (goat) (Bio-Rad Laboratories, Inc.) was used as a secondary antibody. By using TMB One Component HRP Microwell Substrate (SURMODICS), absorbance at 450 nm (control at 630 nm) was measured. The rFC concentration in a sample was calculated by using a calibration curve which has been established in advance using purified FC. Furthermore, for a test in which the commercially available recombinant reagent A is used, the rFC concentration at the time of endotoxin measurement was lower than 162 ng/mL.

### C. Measurement of relative potency of endotoxin of Helicobacter pylori CA2

In a case in which the lysate reagent is used, the relative potency of the endotoxin of Helicobacter pylori CA2 was about 2,000 EU/µg (Table 1). On the basis of this result, it is found to be desirable that, when a recombinant reagent is used, the relative potency of endotoxin of Helicobacter pylori CA2 is 200 EU/µg or higher (Yutaka Kikuchi et al., Pharmaceutical and Medical Device Regulatory Science, Vol. 48 (2017), No. 4, P252-260).

### (1) Endotoxin measuring reagent containing rFC produced with CHO DG44 cells (three factor system)

The relative potency of endotoxin of Helicobacter pylori CA2 was obtained from a three factor system containing rFC, rFB, and rPCE that were produced with CHO DG44 cells.

rFC, rFB, rPCE and a compound for detection (synthetic substrate Boc-LGR-pNA; PEPTIDE INSTITUTE, INC.) were added to Tris buffer solution to prepare an endotoxin measuring reagent. The rFC concentration in each endotoxin measuring reagent was adjusted by varying an addition amount of the enzyme solution of rFC. The measurement reagent was mixed, in a micro plate, with water for injection (blank) or a diluted solution of endotoxin to prepare a measurement sample. Furthermore, the preparation was made such that the concentrations of RSE and endotoxin of Helicobacter pylori CA2 in a measurement sample were 0.025 EU/mL and 0.25 ng/mL, respectively. Measurement of the relative potency was carried out on the basis of Example 1 (commercially available recombinant reagent A). Furthermore, rFB and rPCE were prepared so as to have the same concentration in each measurement sample.

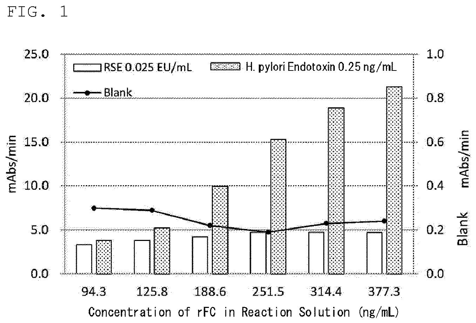

From 2 points between the blank and RSE, a calibration curve was established, and from the absorbance change ratio of endotoxin of Helicobacter pylori CA2, endotoxin activity was measured. The relative potency (EU/µg) of endotoxin of Helicobacter pylori CA2 in the case of using each endotoxin measuring reagent was obtained by dividing the endotoxin activity value (EU/mL), which has been obtained from above, by the concentration of the endotoxin of Helicobacter pylori CA2. As a result of plotting the rFC concentration and the relative potency and performing analysis, the relative potency of the endotoxin of Helicobacter pylori CA2 was 200 EU/µg or higher when the rFC concentration in the measurement sample was 162 ng/mL or more. Namely, it was found that the sensitivity was enhanced by increasing the rFC concentration in a measurement sample (Table 2 and FIG. 1). Furthermore, no increase in the absorbance change ratio was shown from the blank even when rFC concentration in the measurement sample was increased.

**Table 2**

| rFC produced with CHO DG44 cells (three factor system) | | | | |
|---|---|---|---|---|
| rFC | Absorbance change ratio (mAbs/min) | | | Relative potency (EU/µg) |
| ng/mL | Blank | RSE | H. pylori | |
| | | 0.025 EU/mL | 0.25 ng/mL | |
| 94.3 | 0.30 | 3.30 | 3.80 | 117 |
| 125.8 | 0.29 | 3.78 | 5.23 | 141 |
| 188.6 | 0.22 | 4.22 | 9.93 | 242 |
| 251.5 | 0.19 | 4.73 | 15.33 | 333 |
| 314.4 | 0.23 | 4.74 | 18.88 | 414 |
| 377.3 | 0.24 | 4.69 | 21.32 | 473 |

### (2) Endotoxin measuring reagent containing rFC produced with CHO DG44 cells (two factor system)

The relative potency of endotoxin of Helicobacter pylori CA2 was obtained from a two factor system containing rFC and rFB that were produced with CHO DG44 cells (two factor system not containing rPCE).

rFC, rFB, and a compound for detection (synthetic substrate Boc-LTR-MCA (namely, Boc peptidized AMC); PEPTIDE INSTITUTE, INC.) were added to Tris buffer solution to prepare an endotoxin measuring reagent. The rFC concentration in each endotoxin measuring reagent was adjusted by varying an addition amount of the enzyme solution of rFC. The measurement reagent was mixed, in a micro plate, with water for injection (blank) or a diluted solution of endotoxin to prepare a measurement sample. Furthermore, the preparation was made such that the concentrations of RSE and endotoxin of Helicobacter pylori CA2 in a measurement sample were 0.025 EU/mL and 0.25 ng/mL, respectively. Furthermore, rFB was prepared to have the same concentration in each the measurement sample. By using a fluorescence microplate reader, the relative fluorescence intensity of a measurement sample (RFU₀) was measured (excitation wavelength of 380 nm, and fluorescence wavelength of 445 nm). After that, the measurement sample was incubated at 37°C for 90 minutes, and the relative fluorescence intensity (RFU₉₀) was measured again. RFU₀ (background) was subtracted from RFU₉₀ to calculate ΔRFU. From ΔRFU of RSE and the blank, ΔRFU of each blank was subtracted to obtain corrected ΔRFU_{RSE} (Blanked ΔRFU_{RSE}) and corrected ΔRFU_{blank} (Blanked ΔRFU_{blank}) . From two points between corrected ΔRFU_{blank} (that is, 0) and corrected ΔRFU_{RSE} of the blank, a calibration curve was established. Next, from ΔRFU of the endotoxin of Helicobacter pylori CA2, ΔRFU of the blank was subtracted to obtain corrected ΔRFU_{Hpy}, and the endotoxin activity (EU/mL) was obtained from the calibration curve. The relative potency (EU/µg) of endotoxin of Helicobacter pylori CA2 was obtained on the basis of Example 2C.(1) for the case of using each endotoxin measuring reagent.

As a result, when rFC concentration was 235 ng/mL or more in a measurement sample, the relative potency of endotoxin of Helicobacter pylori CA2 was 200 EU/µg or higher. Namely, it was found that the sensitivity was enhanced by increasing the rFC concentration in a measurement sample (Table 3). Furthermore, ΔRFU of the blank was hardly increased even when rFC concentration in the measurement sample was increased.

**Table 3**

| rFC produced with CHO DG44 cells (two factor system) | | | |
|---|---|---|---|
| rFC | Blanked delta RFU | | Relative potency |
| ng/mL | RSE | H. pylori | |
| | 0.025 EU/mL | 0.25 ng/mL | (EU/µg) |
| 94.3 | 582 | 126 | 22 |
| 125.8 | 715 | 428 | 60 |
| 188.6 | 727 | 1090 | 150 |
| 251.5 | 802 | 1750 | 218 |
| 314.4 | 840 | 2497 | 297 |
| 377.3 | 837 | 2982 | 356 |

### (3) Endotoxin measuring reagent containing rFC produced with CHO DG44 cells (one factor system)

The relative potency of endotoxin of Helicobacter pylori CA2 was obtained from a one factor system containing rFC that is produced with CHO DG44 cells (one factor system not containing rFB or rPCE).

rFC and a compound for detection (synthetic substrate Boc-VPR-MCA (namely, Boc peptidized AMC); PEPTIDE INSTITUTE, INC.) were added to Tris buffer solution to prepare an endotoxin measuring reagent. The rFC concentration in each endotoxin measuring reagent was adjusted by varying an addition amount of the enzyme solution of rFC. The measurement reagent was mixed, in a micro plate, with water for injection (blank) or a diluted solution of endotoxin to prepare a measurement sample. Furthermore, the preparation was made such that the concentrations of RSE and endotoxin of Helicobacter pylori CA2 in a measurement sample were 5 EU/mL and 25 ng/mL, respectively. The relative potency (EU/µg) of endotoxin of Helicobacter pylori CA2 was obtained on the basis of Example 2C.(2) for the case of using each endotoxin measuring reagent.

As a result, the relative potency of the endotoxin of Helicobacter pylori CA2 was 200 EU/µg or higher when rFC concentration in the measurement sample was 13 ng/mL or more. Namely, it was found that the sensitivity was enhanced by increasing the rFC concentration in a measurement sample (Table 4). Furthermore, ΔRFU of the blank was hardly increased even when rFC concentration in the measurement sample was increased.

**Table 4**

| rFC produced with CHO DG44 cells (one factor system) | | | |
|---|---|---|---|
| rFC | Blanked delta RFU | | Relative potency (EU/µg) |
| ng/mL | RSE | H. pylori | |
| | 5 EU/mL | 25 ng/mL | |
| 6.3 | 453 | 122 | 54 |
| 9.4 | 656 | 399 | 122 |
| 12.6 | 832 | 754 | 181 |
| 15.7 | 1223 | 1606 | 263 |

### (4) Endotoxin measuring reagent containing rFC produced with Expi CHO Expression System (three factor system)

The relative potency of endotoxin of Helicobacter pylori CA2 was obtained from a three factor system containing rFC, rFB, and rPCE that were produced with Expi CHO Expression System (transient expression system).

The relative potency of endotoxin of Helicobacter pylori CA2 was obtained on the basis of Example 2C.(1). As a result, the relative potency was 200 EU/µg or higher when rFC concentration in the measurement sample was 743 ng/mL or more. Namely, it was found that the sensitivity was enhanced by increasing the rFC concentration in a measurement sample (Table 5). Furthermore, no increase in the absorbance change ratio was shown from the blank even when rFC concentration in the measurement sample was increased.

**Table 5**

| rFC produced with Expi CHO Expression System (three factor system) | | | | |
|---|---|---|---|---|
| rFC | Absorbance change ratio (mAbs/min) | | | Relative potency (EU/µg) |
| ng/mL | Blank | RSE | H. pylori | |
| | | 0.025 EU/mL | 0.25 ng/mL | |
| 669.1 | 0.27 | 4.85 | 8.45 | 178 |
| 836.4 | 0.25 | 4.97 | 11.03 | 228 |
| 1003.7 | 0.24 | 4.84 | 12.23 | 261 |
| 1170.9 | 0.27 | 4.95 | 13.04 | 273 |

### (5) Endotoxin measuring reagent containing rFC produced with Expi CHO Expression System (one factor system)

The relative potency of endotoxin of Helicobacter pylori CA2 was obtained from a one factor system containing rFC that is produced with Expi CHO Expression System (transient expression system) (one factor system not containing rFB or rPCE).

The relative potency of endotoxin of Helicobacter pylori CA2 was obtained on the basis of Example 2C.(3). As a result, the relative potency was 200 EU/µg or higher when rFC concentration in the measurement sample was 77 ng/mL or more. Namely, it was found that the sensitivity was enhanced by increasing the rFC concentration in a measurement sample (Table 6). Furthermore, ΔRFU of the blank was hardly increased even when rFC concentration in the measurement sample was increased.

**Table 6**

| rFC produced with Expi CHO Expression system (one factor system) | | | |
|---|---|---|---|
| rFC | Blanked delta RFU | | Relative potency (EU/µg) |
| ng/mL | RSE | H. pylori | |
| | 5 EU/mL | 25 ng/mL | |
| 16.7 | 653 | 39 | 12 |
| 20.9 | 1053 | 104 | 20 |
| 41.8 | 2374 | 731 | 62 |
| 83.6 | 4355 | 4925 | 226 |

### (6) Endotoxin measuring reagent containing rFC produced with Sf9 cells (three factor system)

The relative potency of endotoxin of Helicobacter pylori CA2 was obtained from a three factor system containing rFC, rFB, and rPCE that were produced with Sf9 cells.

The relative potency of endotoxin of Helicobacter pylori CA2 was obtained on the basis of Example 2C.(1). As a result, the relative potency was 200 EU/µg or higher when rFC concentration in the measurement sample was 394 ng/mL or more. Namely, it was found that the sensitivity was enhanced by increasing the rFC concentration in a measurement sample (Table 7). Furthermore, no increase in the absorbance change ratio was shown from the blank even when rFC concentration in the measurement sample was increased.

**Table 7**

| rFC produced with Sf9 (three factor system) | | | | |
|---|---|---|---|---|
| rFC | Absorbance change ratio (mAbs/min) | | | Relative potency (EU/µg) |
| ng/mL | Blank | RSE | H. pylori | |
| | | 0.025 EU/mL | 0.25 ng/mL | |
| 185.5 | 0.38 | 5.56 | 3.96 | 69 |
| 247.4 | 0.35 | 5.25 | 5.35 | 102 |
| 309.2 | 0.35 | 4.78 | 6.37 | 136 |
| 371.0 | 0.36 | 3.86 | 6.04 | 162 |
| 495.1 | 0.38 | 2.75 | 8.99 | 363 |
| 618.9 | 0.38 | 1.84 | 9.16 | 601 |

### (7) Endotoxin measuring reagent containing rFC produced with Sf9 cells (one factor system)

The relative potency of endotoxin of Helicobacter pylori CA2 was obtained from a one factor system containing rFC that is produced with Sf9 cells (one factor system neither containing rFB nor rPCE).

The relative potency of endotoxin of Helicobacter pylori CA2 was obtained on the basis of Example 2C.(3). Here, the preparation was made such that the concentrations of RSE and endotoxin of Helicobacter pylori CA2 in a measurement sample were 0.25 EU/mL and 0.25 ng/mL, respectively. As a result, the relative potency was 200 EU/µg or higher when rFC concentration in the measurement sample was 93 ng/mL or more. Namely, it was found that the sensitivity was enhanced by increasing the rFC concentration in a measurement sample (Table 8). Furthermore, ΔRFU of the blank was hardly increased even when rFC concentration in the measurement sample was increased.

**Table 8**

| rFC produced with Sf9 (one factor system) | | | |
|---|---|---|---|
| rFC | Blanked delta RFC | | Relative potency (EU/µg) |
| ng/mL | RSE | H. pylori | |
| | 0.25 EU/mL | 0.25 ng/mL | |
| 65.0 | 469 | 83 | 177 |
| 86.7 | 527 | 99 | 188 |
| 130.0 | 640 | 177 | 277 |
| 173.3 | 789 | 287 | 364 |
| 216.7 | 873 | 450 | 515 |
| 260.0 | 904 | 602 | 666 |

### <Example 3: Influence of rFC content on sensitivity to endotoxin of Helicobacter pylori CA2>

### A. Preparation of recombinant protein

On the basis of Example 2A.(1), rFC of Limulus Polyphemus, L.p. was expressed using Chinese hamster ovarian cell line (CHO DG44 cells) as a host to obtain an enzyme solution of L.p. rFC. Furthermore, SEQ ID NO: 11 was used as DNA encoding FC of L.p.

### B. Measurement of L.p. rFC concentration

The L.p. rFC concentration was measured on the basis of Example 2B. The L.p. rFC concentration in a sample was calculated by using a calibration curve which has been established in advance using purified L.p. rFC.

### C. Measurement of relative potency of endotoxin of Helicobacter pylori CA2

### (1) Endotoxin measuring reagent containing L.p. rFC produced with CHO DG44 cells (one factor system)

The relative potency of endotoxin of Helicobacter pylori CA2 was obtained from a one factor system containing L.p. rFC that was produced with CHO DG44 cells (one factor system not containing rFB or rPCE). Furthermore, the preparation was made such that the concentrations of RSE and endotoxin of Helicobacter pylori CA2 in a measurement sample were 5 EU/mL and 25 ng/mL, respectively.

The relative potency (EU/µg) of endotoxin of Helicobacter pylori CA2 was obtained on the basis of Example 2C.(3). When the L.p. rFC concentration in a reaction solution was gradually increased from 45.8 ng/mL to 457.9 ng/mL, the relative potency (EU/µg) of endotoxin of Helicobacter pylori CA2 was increased from 33 EU/µg to 917 EU/µg. As a result of plotting the rFC concentration and the relative potency and performing analysis, the relative potency of the endotoxin of Helicobacter pylori CA2 was 200 EU/µg or higher when the L.p. rFC concentration in the measurement sample was 133 ng/mL or more. Namely, also in measurement of one factor system using L.p. rFC, it was found that the sensitivity is enhanced by increasing the L.p. rFC concentration in a measurement sample.

### <Example 4: Influence of rFC content on sensitivity to endotoxin of Helicobacter pylori CA2>

As rFC, rFC Enzyme Solution contained in the commercially available recombinant reagent B (PyroGene, LONZA JAPAN) was used.

### A. Measurement of rFC concentration in rFC Enzyme Solution

The rFC concentration was measured on the basis of Example 2B. The rFC concentration in a sample was calculated by using a calibration curve which had been established in advance using purified Carcinoscorpius rotundicauda, C.r.) rFC.

### B. Measurement of relative potency of endotoxin of Helicobacter pylori CA2

### (1) Endotoxin measuring reagent containing rFC (one factor system)

The relative potency of endotoxin of Helicobacter pylori CA2 was obtained from a one factor system using rFC Enzyme Solution (one factor system not containing rFB or rPCE). Furthermore, the preparation was made such that the concentrations of RSE and endotoxin of Helicobacter pylori CA2 in a measurement sample were 0.025 EU/mL and 5 ng/mL, respectively.

The relative potency (EU/µg) of endotoxin of Helicobacter pylori CA2 was obtained on the basis of Example 2C.(3). When the rFC concentration in a reaction solution was gradually increased from 203.7 ng/mL to 1,426 ng/mL, the relative potency (EU/µg) of endotoxin of Helicobacter pylori CA2 was increased from 9.09 EU/µg to 440 EU/µg. As a result of plotting the rFC concentration and the relative potency and performing analysis, the relative potency of the endotoxin of Helicobacter pylori CA2 was 200 EU/µg or higher when the rFC concentration in the measurement sample was 858 ng/mL or more. Namely, it was found that the sensitivity was enhanced by increasing the rFC concentration in a measurement sample even in the case of using a commercially available recombinant reagent.

### <Reference Example 1: Influence of rFB content on sensitivity to endotoxin of Helicobacter pylori CA2>

A measurement sample having a different rFB content was prepared, and the relative potency of endotoxin of Helicobacter pylori CA2 was obtained on the basis of Example 2C.(1). Furthermore, the preparation was made such that rFC and rPCE had the same concentrations in each measurement sample.

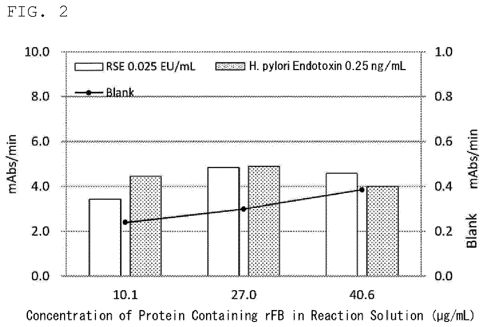

As a result, when the rFB concentration in a measurement sample is increased, the absorbance change ratio was increased in the blank and RSE, but the absorbance change ratio of endotoxin of Helicobacter pylori CA2 was hardly changed. Namely, it was found that the sensitivity to endotoxin of Helicobacter pylori CA2 was not enhanced even when the rFB concentration in a measurement sample was increased (FIG. 2).

### <Reference Example 2: Influence of rPCE content on sensitivity to endotoxin of Helicobacter pylori CA2>

A measurement sample having a different rPCE content was prepared, and relative potency of endotoxin of Helicobacter pylori CA2 was obtained on the basis of Example 2C.(1). Furthermore, the preparation was made such that rFC and rFB had the same concentrations in each measurement sample.

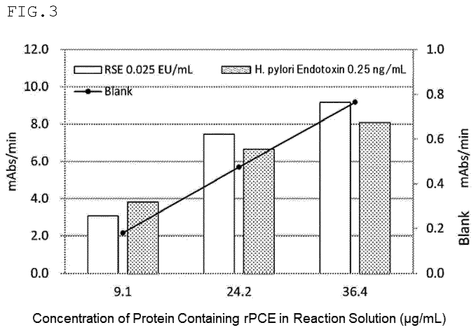

As a result, when the rPCE concentration in the measurement sample was increased, the absorbance change ratio was simultaneously increased in the blank, RSE, and endotoxin of Helicobacter pylori CA2. Namely, it was found that the sensitivity to endotoxin of Helicobacter pylori CA2 was not enhanced even when the rPCE concentration in a measurement sample was increased (FIG. 3).

### Industrial Applicability

According to the present invention, a method for enhancing the sensitivity of an endotoxin measuring reagent employing a recombinant protein to the endotoxin of Helicobacter pylori can be provided. Accordingly, the present invention is to provide an endotoxin measuring method having reduced influence caused by a difference in origin of endotoxin in which an endotoxin measuring reagent employing a recombinant protein is used.

### <Description of Sequence Listing>

SEQ ID NO: 1: cDNA base sequence of factor C of Tachypleus tridentatus
SEQ ID NO: 2: Amino acid sequence of factor C of Tachypleus tridentatus
SEQ ID NO: 3: cDNA base sequence of factor C of Carcinoscorpius rotundicauda
SEQ ID NO: 4: Amino acid sequence of factor C of Carcinoscorpius rotundicauda
SEQ ID NO: 5: cDNA base sequence of factor B of Tachypleus tridentatus
SEQ ID NO: 6: Amino acid sequence of factor B of Tachypleus tridentatus
SEQ ID NO: 7: cDNA base sequence of pro-clotting enzyme of Tachypleus tridentatus
SEQ ID NO: 8: Amino acid sequence of pro-clotting enzyme of Tachypleus tridentatus
SEQ ID NO: 9: cDNA base sequence of factor B of Tachypleus tridentatus in which codons are optimized for expression in insect cells
SEQ ID NO: 10: cDNA base sequence of factor C of Limulus polyphemus
SEQ ID NO: 11: cDNA base sequence of factor C of Limulus polyphemus in which codons are optimized for expression in CHO cells
SEQ ID NO: 12: Amino acid sequence of factor C of Limulus polyphemus

This application claims priority from Japanese Patent Application No. 2018-188587 filed October 3, 2018, the entire contents of which are hereby incorporated by reference herein.

## Claims

1. A method for enhancing a sensitivity of an endotoxin measuring reagent to the endotoxin of Helicobacter pylori,
the endotoxin measuring reagent comprising a recombinant protein of horseshoe crab factor C,
the method comprising increasing a content of the recombinant protein of factor C at the time of endotoxin measurement.

2. The method according to claim 1, wherein the endotoxin measuring reagent comprises a recombinant protein of horseshoe crab factor B.

3. The method according to claim 1 or 2, wherein the endotoxin measuring reagent comprises a recombinant protein of horseshoe crab pro-clotting enzyme.

4. The method according to any one of claims 1 to 3, wherein the endotoxin measuring reagent comprises a compound for detection.

5. An endotoxin measuring method in which sensitivity of an endotoxin measuring reagent to the endotoxin of Helicobacter pylori is enhanced, the method comprising:
preparing an endotoxin measurement sample comprising the endotoxin measuring reagent and an analyte,
wherein the endotoxin measuring reagent comprises a recombinant protein of horseshoe crab factor C, and
the sensitivity is enhanced by increasing a concentration of the recombinant protein of factor C in the endotoxin measurement sample.

6. The method according to claim 5, wherein the endotoxin measuring reagent comprises a recombinant protein of horseshoe crab factor B.

7. The method according to claim 5 or 6, wherein the endotoxin measuring reagent comprises a recombinant protein of horseshoe crab pro-clotting enzyme.

8. The method according to any one of claims 5 to 7, wherein the endotoxin measuring reagent comprises a compound for detection.

9. A method for producing an endotoxin measuring reagent having enhanced sensitivity to the endotoxin of Helicobacter pylori,
the endotoxin measuring reagent comprising a recombinant protein of horseshoe crab factor C,
the method comprising increasing a content of the recombinant protein of factor C in the endotoxin measuring reagent.

10. An endotoxin measuring reagent comprising a horseshoe crab factor C,
wherein the horseshoe crab factor C is a recombinant protein, and
relative potency of endotoxin of Helicobacter pylori is 200 (EU/µg) or more.

11. The endotoxin measuring reagent according to claim 10, comprising a recombinant protein of horseshoe crab factor B, a recombinant protein of horseshoe crab pro-clotting enzyme, and a compound for detection represented by general formula Y-X-Z,
wherein in the above general formula, Y is a hydrogen atom or a protecting group, X is a peptide comprising the amino acid sequence, which is a substrate of the recombinant protein of pro-clotting enzyme, and Z is a labeling material which becomes optically detectable when being released from X.

12. The endotoxin measuring reagent according to claim 10, comprising a recombinant protein of horseshoe crab factor B and a compound for detection represented by general formula Y-X-Z,
wherein in the above general formula, Y is a hydrogen atom or a protecting group, X is a peptide comprising the amino acid sequence, which is a substrate of the recombinant protein of factor B, and Z is a labeling material which becomes optically detectable when being released from X.

13. The endotoxin measuring reagent according to claim 10, comprising a compound for detection represented by general formula Y-X-Z,
wherein in the above general formula, Y is a hydrogen atom or a protecting group, X is a peptide comprising the amino acid sequence, which is a substrate of the factor C, and Z is a labeling material which becomes optically detectable when being released from X.
